# EUROPÄISCHE PATENTSCHRIFT

(11) **EP 3 881 803 B1**
(45) Veröffentlichungstag und Bekanntmachung des Hinweises auf die Patenterteilung: **29.06.2022**
(21) Anmeldenummer: 20164534.8
(22) Anmeldetag: 20.03.2020
(51) Int. Cl.: A61F 2/32, A61F 2/40, A61F 2/30, A61F 2/36

(54) **VORRICHTUNG UND VERFAHREN ZUR HERSTELLUNG VON SPACERN**
DEVICE AND METHOD FOR PRODUCING SPACERS
DISPOSITIF ET PROCÉDÉ DE FABRICATION D'ESPACEURS

(43) Veröffentlichungstag der Anmeldung: 22.09.2021
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(56) Entgegenhaltungen:
- EP-A1- 2 787 928
- EP-A1- 3 245 981
- WO-A1-2017/125832
- DE-A1-102017 107 569
- US-A1- 2009 175 978
- US-B2- 7 789 646

## Beschreibung

Die Erfindung betrifft eine Vorrichtung zum Herstellen eines Spacers durch Aushärten von Knochenzementteig. Der Spacer ist als temporärer Platzhalter dazu vorgesehen, im medizinischen Anwendungsbereich ein Gelenk oder einen Teil eines Gelenks umfassend eine artikulierende Oberfläche des Gelenks temporär zu ersetzen. Bevorzugt ist der Spacer zum temporären Ersetzen eines Hüftgelenks oder eines Schultergelenks geeignet und vorgesehen. Dementsprechend ist die Vorrichtung bevorzugt zum Herstellen eines Hüftgelenkspacers oder eines Schultergelenkspacers vorgesehen. Die Erfindung betrifft auch ein Verfahren zur Herstellung eines solchen Spacers mit einer solchen Vorrichtung.

Gelenk-Endoprothesen, wie Hüftgelenk-Endoprothesen und Schultergelenk-Endoprothesen, werden in großem Umfang weltweit implantiert. Leider kommt es in einem geringen Prozentsatz vor, dass Gelenk-Endoprothesen von mikrobiellen Keimen, besonders Grampositiven Bakterien als auch Gram-negativen Bakterien und in sehr geringem Umfang von Hefen und Pilzen, besiedelt werden. Diese mikrobiellen Keime, hauptsächlich typische Hautkeime wie Staphylococcus aureus und Staphylococcus epidermidis, können während einer chirurgischen Operation (OP) in den Patienten gelangen. Daneben ist es auch möglich, dass mikrobielle Keime hämatogen Gelenk-Endoprothesen erreichen. Bei einer Besiedlung von Gelenk-Endoprothesen mit mikrobiellen Keimen wird das umliegende Knochen- und Weichgewebe mit infiziert und von den mikrobiellen Keimen geschädigt.

Stand der Technik sind vor allem zwei Behandlungsverfahren für infizierte Gelenk-Endoprothesen, die einzeitige septische Revision und die zweizeitige septische Revision. Bei der einzeitigen Revision wird innerhalb einer OP zuerst die infizierte Gelenk-Endoprothesen entfernt, anschließend radikal debridiert und dann wird eine Revisions-Gelenk-Endoprothese implantiert.

Bei zweizeitigen septischen Revisionen wird in einer ersten OP zuerst die infizierte Gelenk-Endoprothese entfernt, dann wird debridiert und anschließend erfolgt die Implantation eines Spacers. Ein Hüftgelenkspacer besteht aus einem Schaft, einem Kragen, einem Hals und einem Kugelkopf und ist den Hüftgelenk-Endoprothesen in Form und Größe nachgebildet. Analog ist ein Schultergelenkspacer einer Schultergelenk-Endoprothesen in Form und Größe nachgebildet. Der Spacer wird mit Knochenzement an dem jeweiligen Knochen verankert, also beispielsweise bei Hüftgelenkspacern am proximalen Femur beziehungsweise im Femurkanal verankert. Der Spacer verbleibt bis zu mehreren Wochen im Patienten bis die Entzündung abgeklungen ist und die klinischen Entzündungsmarker zurückgegangen sind. Dann wird in einer zweiten OP der Spacer entfernt und nach erneutem Debridement eine Revisions-Gelenk-Endoprothese implantiert.

Bei Spacern werden dem Zementpulver vor der eigentlichen Spacerherstellung Antibiotika zugesetzt. Mit diesem antibiotisch modifizierten Knochenzementpulver wird anschließend durch Beimischen von Monomerflüssigkeit ein Knochenzementteig hergestellt und aus diesem Knochenzementteig werden Spacer gegossen, die dann durch Polymerisation mit Hilfe der dem Zementpulver zugesetzten Monomerflüssigkeit aushärten. Der Knochenzementteig schließt dabei die Antibiotika im Wesentlichen ein. Die in den oberflächennahen Bereichen befindlichen Antibiotika-Partikel werden bei Einwirkung von Körperflüssigkeiten, wie Wundsekret, herausgelöst. Die Wirkstofffreisetzung ist initial am höchsten und nimmt dann im Verlauf von mehreren Tagen ab.

Die US 2010/0042213 A1 offenbart eine Hüftgelenkprothese mit einem Reservoir einer Flüssigkeit im Inneren des Implantats. Aus der WO 2017/178951 A1 ist ein Hüftspacer mit Vertiefungen bekannt, wobei in den Vertiefungen eine Substanz zur Behandlung des Knochens eingebracht werden kann. In dem Patent US 6 245 111 B1 wird eine Hüftgelenksprothese vorgeschlagen, deren Oberflächen mit einem Antibiotikum beschichtet sind. Das Patent US 5 681 289 offenbart eine Einrichtung zum Verteilen eines flüssigen Wirkstoffs mit Hilfe einer Blase im Inneren der Einrichtung. Keine der genannten Prothesen ist zum Erzeugen eines Spülkreislaufs geeignet. In der EP 1 991 170 B1 und der US 2011/0015754 A1 werden ein Hüftgelenkspacer beschrieben, die Wirkstoffe enthalten. Die US 2019/0290833 A1 offenbart einen spülbaren Hüftgelenkspacer, mit dem ein Flüssigkeitskreislauf erzeugbar ist. In der WO 2016/205077 A1 und der US 8 900 322 B2 werden weitere Spacer mit einer Spülfunktion beschrieben.

Es ist bekannt, mit Antibiotika ausgerüstete Spacer zu verwenden. Spacer können einerseits vom OP-Personal während der OP selbst aus PMMA-Knochenzementpulver, Antibiotika und Monomerflüssigkeit hergestellt werden, zum Beispiel mit einer Spacerform, wie sie beispielsweise in den Patenten DE 10 2015 104 704 B4 oder EP 2 617 393 B1 beschrieben sind, und andererseits ist es auch üblich, industriell aus Knochenzement vorgefertigte Hüftgelenkspacer einzusetzen. Kunststoffgießformen zur intraoperativen Herstellung von einteiligen Hüftspacern wurden US 6 361 731 B1 beschrieben. Diese Gießformen sind transparent und haben zwei separate Einfüllöffnungen. Dadurch kann auch hochviskoser Knochenzementteig mit geringem Druck in die Gießform eingebracht werden, weil die Fließwege des Knochenzementteigs relativ kurz sind. Bei der Verwendung von nicht hochviskosem Knochenzementteig besteht die Gefahr, dass Knochenzementteig nach erfolgter Füllung der Gießform vor der einsetzenden Aushärtung wieder aus den Einfüllöffnungen herausfließt.

In einer Weiterentwicklung wurden in den Patentschriften US 7 637 729 B2, US 7 789 646 B2, US 8 480 389 B2 und US 8 801 983 B2 mehrteilige Gießformen für die Herstellung modularer Hüftspacer vorgeschlagen. Diese modularen Hüftspacer bestehen aus einem Spacerkopf und einem separaten Schaft. Daher sind eine Gießform für den Spacerkopf und eine separate Gießform für den Schaft notwendig. Die Gießform für den Schaft ist einteilig und besitzt an der Einfüllöffnung ein Gewinde zur Verbindung der Gießform mit einer Zementkartusche enthaltend den Knochenzementteig.

In der US 2007/0222114 A1 wird eine Hüftspacerform beschrieben. Diese Spacerform besteht aus einer Vielzahl von Formsegmenten, die miteinander verbunden werden. Durch die Vielzahl der Segmente kann die Spacerform recht genau an die anatomischen Gegebenheiten des Patienten angepasst werden. Die Spacerform-Segmente werden mittels Schraubschellen aneinandergefügt. Durch Kanäle in der Spacerform wird ein PMMA-Knochenzementteig (Polymethylmethacrylat-Knochenzementteig) eingeführt. Durch den komplexen Aufbau der Gießform ist es sehr aufwändig, die Spacerform-Segmente zusammenzufügen und nach erfolgter Aushärtung des PMMA-Knochenzementteigs den Hüftspacer zu entnehmen.

In der WO 2009/073 781 A2 wird eine Spacerform für einen Hüftspacer vorgeschlagen, die aus zwei Teilen besteht, die in Bezug zueinander verschoben werden können, um die Länge des Schafts anpassen zu können. Eine weitere Gießform ist in der EP 2 522 310 A1 offenbart. Diese Vorrichtung besteht aus wenigstens zwei Teilen, wobei in einem ersten Teil eine Einschubabschnitt und im zweiten Teil eine Einschubaufnahme angeordnet sind. Beide Teile sind in einander steckbar und bilden eine Gießform für die Herstellung des Schafts des Hüftspacers. In der EP 2 787 928 A1 wird eine komplexe Gießform beschrieben. Diese ermöglicht die Herstellung von Hüftspacern mit unterschiedlichen Kugelköpfen. Die Fixierung der Elemente der Gießform erfolgt über Verbindungselemente. Im Patent US 7 789 646 B2 wird eine Gießform beschrieben, bei der mit einem Stöpsel die Einfüllöffnung der Gießform verschlossen werden kann, wenn der Knochenzementteig in die Gießform eingebracht wurde. Zuvor muss jedoch die Gießform von der Knochenzementkartusche abgeschraubt werden. Bei Verwendung von nicht hochviskosem Knochenzementteig ist es daher möglich, wenn die Gießform ungünstig gehalten wird, dass Knochenzementteig während der Trennung der Gießform von der Knochenzementkartusche ausläuft, bevor der Stöpsel eingeschraubt beziehungsweise eingesteckt ist.

Die Aufgabe der vorliegenden Erfindung besteht also darin, die Nachteile des Stands der Technik zu überwinden. Insbesondere besteht die Aufgabe der Erfindung in der Entwicklung einer kostengünstigen Vorrichtung zum Herstellen eines Spacers durch Aushärten von Knochenzementteig und in der Entwicklung eines einfach und kostengünstig durchführbaren Verfahrens zum Herstellen eines Spacers durch Aushärten von Knochenzementteig, mit dem vom medizinischen Personal im OP unter Verwendung von Knochenzementteig, insbesondere von Polymethylmethacrylat-Knochenzement, einteilige Spacer, insbesondere Hüft- und Schulterspacer, hergestellt werden können. Hüft- und Schulterspacer sind ähnlich aufgebaut. Sie bestehen aus einem Schaft und einem Spacerkopf. Ein Metallkern kann zur mechanischen Stabilisierung im Inneren des Hüft- und Schulterspacer angeordnet sein beziehungsweise werden. Es soll möglich sein, sowohl mit (Polymethylmethacrylat-)Knochenzementteig niedriger beziehungsweise nicht hoher Viskosität als auch mit hoher Viskosität Spacer herzustellen. Für die vollständige Füllung einer Gießform mit einem hochviskosem Knochenzementteig ist ein hoher Einpressdruck erforderlich. Die Gießform der Vorrichtung soll dem hohen Einpressdruck widerstehen. Die Gießform soll daher möglichst einem Druck von 10 N/cm² standhalten.

Die Vorrichtung soll so beschaffen sein, dass Knochenzementteig beziehungsweise fluider Knochenzementteig aus einer Knochenzementkartusche in eine Gießform eingepresst werden kann. Bei Verwendung von nicht hochviskosem Knochenzementteig soll nach Füllung der Gießform der Knochenzementteig nicht aus der Gießform ausfließen. Dazu ist es notwendig, die Gießform so zu gestalten, dass ein Auslaufen von nicht hochviskosem Knochenzementteig aus der Gießform während der Trennung der Gießform von der Zementkartusche sicher verhindert wird. Dieses Verschließen soll möglich sein, ohne dass in der Wandung der Gießform Öffnungen für komplex aufgebaute Ventile notwendig sind. Öffnungen in der Wandung der Gießform können zur Undichtigkeit der Gießform führen, wenn der Knochenzementteig unter hohem Druck in die Gießform eingepresst wird. Weiterhin soll der Angussbereich der Gießform so gestaltet sein, dass einerseits eine leichte Befüllung der Gießform mit Knochenzementteig möglich ist und dass andererseits nach erfolgter Aushärtung des Knochenzementteigs die Angussreste leicht entfernt werden können.

Die Aufgaben der Erfindung werden gelöst durch eine Vorrichtung zum Herstellen eines Spacers durch Aushärten von Knochenzementteig, wobei der Spacer dazu vorgesehen ist, im medizinischen Bereich ein Gelenk oder einen Teil eines Gelenks umfassend eine artikulierende Oberfläche des Gelenks temporär zu ersetzen, insbesondere ein Hüftgelenk oder ein Schultergelenk temporär zu ersetzen, die Vorrichtung aufweisend
A) eine Gießform zum Formen des Spacers aus Knochenzementteig mit wenigstens einer Einfüllöffnung zum Einfüllen eines Knochenzementteigs;
B) einen Ventilsitz, der im Bereich der wenigstens einen Einfüllöffnung mit der Gießform verbunden ist, wobei der Ventilsitz eine bereichsweise geschlossene Kopfseite mit mindestens einer ersten Durchführung aufweist, wobei die mindestens eine erste Durchführung in die wenigstens eine Einfüllöffnung mündet;
C) einen Ventilkörper, der drehbar gegen den Ventilsitz gelagert ist und der eine Dichtfläche aufweist, wobei die Dichtfläche in Richtung der bereichsweise geschlossenen Kopfseite des Ventilsitzes ausgerichtet ist, wobei in der Dichtfläche mindestens eine zweite Durchführung angeordnet ist;
wobei der Ventilsitz und der Ventilkörper zusammen ein Ventil bilden, wobei das Ventil durch Drehen des Ventilkörpers gegen den Ventilsitz reversibel in eine geöffnete Stellung und reversibel in eine geschlossene Stellung überführbar ist, wobei in der geöffneten Stellung des Ventils die mindestens eine erste Durchführung des Ventilsitzes und die mindestens eine zweite Durchführung des Ventilkörpers zumindest bereichsweise übereinanderliegen und eine für Knochenzementteig durchlässige Verbindung durch das Ventil in die Gießform bereitstellen, wobei in der geschlossenen Stellung des Ventils die mindestens eine erste Durchführung des Ventilsitzes durch die Dichtfläche des Ventilkörpers abgedeckt ist, wobei die wenigstens eine Einfüllöffnung der Gießform in der geschlossenen Stellung des Ventils für Knochenzementteig abgedeckt ist und wobei das Ventil auf der von der Gießform abgewandten Seite mit einem Anschluss zum flüssigkeitsdichten Verbinden einer Knochenzementkartusche verbunden ist oder das Ventil einen solchen Anschluss aufweist.

Flüssigkeitsdicht bedeutet, dass der nicht ausgehärtete also fluide Knochenzementteig und bevorzugt auch eine flüssige Monomerflüssigkeit als Ausgangskomponente des Knochenzements nicht durch die Verbindung am Anschluss ausfließen oder hindurchdringen können.

Für Knochenzementteig abgedeckt bedeutet, dass der Knochenzementteig im Ventil zumindest derart stark am Fließen gehindert wird, dass er nicht durch das Ventil hindurchfließen kann, bevor er aushärtet. Hierzu reicht es für normal viskose Knochenzementteige bereits aus, wenn der Knochenzementteig nicht in gerader Linie durch das Ventil strömen kann und die freien Leitungsquerschnitte kleiner als 1 mm sind. Knochenzementteige sind viskose oder hochviskose Fluide, wie durch den Zusatz "Teig" angedeutet. Die Viskosität eines Knochenzementteigs beträgt mindestens 10 Pa s, was der Viskosität von flüssigem Honig entspricht. Zudem härtet der Knochenzementteig innerhalb von wenigen Minuten aus, so dass anschließend kein Durchtritt mehr möglich ist. Es kann bevorzugt vorgesehen sein, dass der Knochenzementteig eine Viskosität von mindestens 10 Pa s aufweist.

Unter einem Knochenzementteig beziehungsweise einem fluiden Knochenzementteig ist ein gemischter (also zum Einsatz bereiter) Knochenzementteig zu verstehen, der eine zähflüssige Konsistenz hat. Die Viskosität eines Knochenzements entspricht vorzugsweise der von Honig oder hat eine noch zähflüssigere, das heiß höhere Viskosität. Die Begriffe fluider Knochenzement und Knochenzementteig werden synonym verwendet.

Bevorzugt ist die Gießform innen hohl.

Es kann vorgesehen sein, dass die Dichtfläche bis auf die mindestens eine zweite Durchführung geschlossen ist.

Es kann bevorzugt auch vorgesehen sein, dass die Gießform einen Innenraum aufweist, der eine Negativform eines Gelenkkopfs nachbildet, insbesondere eines Hüftgelenkkopfs oder Schultergelenkkopfs nachbildet.

Es kann auch vorgesehen sein, dass der Ventilsitz flüssigkeitsundurchlässig mit einer Gießformwand der Gießform verbunden ist.

Ferner kann vorgesehen sein, dass der Ventilsitz an einer Stirnseite eines durch die Gießform begrenzten Hohlraums als Scheibe ausgebildet ist, insbesondere als ebene Scheibe ausgebildet ist.

Bevorzugt kann auch vorgesehen sein, dass der Ventilsitz und der Ventilkörper hohlzylinderförmig sind.

Es kann ferner vorgesehen sein, dass die Gießform zweiteilig oder mehrteilig ist, wobei vorzugsweise die Teile der Gießform über Flansche flüssigkeitsdicht aneinander befestigbar sind. Besonders bevorzugt ist die Gießform zweiteilig.

Die Gießform soll einem Druck von 10 N/cm² standhalten, um auch hochviskosen Knochenzementteig verwenden zu können.

Die Begriffe "geöffneter Zustand" und "geschlossener Zustand" des Ventils beziehungsweise des Ventilkörpers relativ zum Ventilsitz und die Begriffe "geöffnete Stellung" und "geschlossene Stellung" des Ventils beziehungsweise des Ventilkörpers relativ zum Ventilsitz werden synonym verwendet.

In der vorliegenden Patentanmeldung werden die Richtungsbezeichnungen "proximal", "distal" und "lateral" sowie die Ebenen-bezeichnungen "Sagittalebene", "Frontalebene" und "Transversalebene" in Bezug auf den Spacer oder die Gießform so verwendet, wie dies bei dem im Patienten eingesetzten Zustand als anatomische Hauptrichtung oder als Körperebene zu verstehen wäre. Dabei bedeutet "proximal" zum Körperzentrum hin und "distal" vom Körperzentrum entfernt.

Der Schaft ist zur Verbindung mit einem Knochen (im Fall von Hüftgelenkspacern mit dem Femur) vorgesehen und ist vorzugsweise zu diesem Zweck in ein proximales Ende des präparierten Knochens beziehungsweise in den Knochenkanal einführbar.

Bevorzugt kann vorgesehen sein, dass die Vorrichtung zur Herstellung eines Spacers, insbesondere eines Hüftgelenkspacers oder Schultergelenkspacers, zur Anwendung zumindest eines antibiotischen und/oder antimykotischen Wirkstoffs geeignet ist.

Bevorzugt ist der Spacer einteilig aus einem biokompatiblen Knochenzementteig, wie Polymethylmethacrylat (PMMA), zu fertigen, wobei besonders bevorzugt das PMMA wenigsten ein aus dem PMMA lösbares Antibiotikum und/oder Antimykotikum enthält.

Es kann bevorzugt vorgesehen sein, dass die bereichsweise geschlossene Kopfseite des Ventilsitzes und die Dichtfläche des Ventilkörpers Scheiben sind oder scheibenförmig sind.

Es kann bevorzugt vorgesehen sein, dass der Ventilsitz die Einfüllöffnung der Gießform begrenzt.

Es kann vorgesehen sein, dass der Ventilsitz nicht verdrehbar gegen die Gießform mit der Gießform verbunden ist, bevorzugt der Ventilsitz fest und/oder starr mit der Gießform verbunden ist.

Hierdurch kann auf konstruktiv einfache und damit kostengünstige Weise eine Abdichtung beziehungsweise Verbindung des Ventilsitzes für den Knochenzementteig gegen die Gießform erreicht werden.

Ferner kann vorgesehen sein, dass das Ventil manuell bedienbar ist, bevorzugt manuell von außerhalb der Vorrichtung manuell bedienbar ist, wobei besonders bevorzugt der Ventilkörper manuell gegen den Ventilsitz drehbar ist und durch die Drehung das Ventil von der geschlossenen Stellung in die geöffnete Stellung und von der geöffneten Stellung in die geschlossene Stellung überführbar ist.

Hierdurch kann das Ventil der Vorrichtung bequem von außen bedient werden, um eine Knochenzementkartusche zu wechseln oder diese zu lösen.

Es kann auch vorgesehen sein, dass das Ventil durch Drehen oder Kippen einer an den Anschluss angeschlossenen Zementkartusche bedienbar ist, wobei hierzu vorzugsweise der Anschluss an dem Ventilkörper angeordnet ist.

Des Weiteren kann vorgesehen sein, dass die mindestens eine erste Durchführung des Ventilsitzes in der geschlossenen Stellung des Ventils mit der Dichtfläche des Ventilkörpers abgedeckt ist, wobei bevorzugt die bereichsweise geschlossene Kopfseite des Ventilsitzes und die Dichtfläche des Ventilkörpers maximal 2 mm voneinander beabstandet sind, besonders bevorzugt maximal 1 mm voneinander beabstandet sind, ganz besonders bevorzugt maximal 0,5 mm voneinander beabstandet sind.

Hierdurch kann sichergestellt werden, dass der in die Gießform eingefüllte Knochenzementteig (der fluide Knochenzement) nicht wieder aus der Gießform durch das Ventil herausgedrückt werden kann, wenn das Ventil geschlossen ist. Wenn der Knochenzementteig mit diesen Dicken beziehungsweise mit diesen Querschnitten im Bereich des Angusses aushärtet, kann er nach dem Aushärten des Spacers leicht manuell abgebrochen oder durchgeschnitten werden und muss nicht mit einer Säge aufgetrennt werden. Daher sind Angüsse mit solchen Dicken unschädlich, da sie den OP-Betrieb während einer OP nicht nennenswert aufhalten.

Es kann auch vorgesehen sein, dass der Ventilkörper um eine Drehachse gegen den Ventilsitz drehbar gelagert ist, wobei die Drehachse senkrecht zur Dichtfläche des Ventilkörpers verläuft oder wobei die Drehachse entlang einer Rotationssymmetrieachse der Dichtfläche des Ventilkörpers verläuft.

Hiermit wird erreicht, dass der durch das Ventil fließende Knochenzementteig von dem Ventilkörper durch die Drehung abgeschnitten beziehungsweise abgedreht werden kann. Dies ermöglicht eine glatte Schnittfläche und einen geringen Kraftaufwand beim Abscheren. Ferner kann auch eine Drehung der Knochenzementkartusche zum Abscheren des Knochenzementteigs verwendet werden. Es wird bevorzugt, dass die Drehachse entlang der Rotationssymmetrieachse der Dichtfläche des Ventilkörpers verläuft. Wenn die Drehachse senkrecht zur Dichtfläche des Ventilkörpers verläuft, kann das Ventil nach Art eines Zapfhahns (beispielsweise für Bier) aufgebaut sein.

Es kann auch vorgesehen sein, dass der Ventilkörper um eine Drehachse gegen den Ventilsitz drehbar gelagert ist, wobei die Drehachse in Richtung der Einfüllöffnung ausgerichtet ist.

Es kann ferner vorgesehen sein, dass sich der Ventilkörper um einen Winkel von maximal 280° gegen den Ventilsitz verdrehen lässt, bevorzugt von maximal 180°, besonders bevorzugt von maximal 100° gegen den Ventilsitz verdrehen lässt, ganz besonders bevorzugt von bis zu 90° gegen den Ventilsitz verdrehen lässt.

Bevorzugt können jeweils zwei Durchführungen in dem Ventilsitz und in dem Ventilkörper angeordnet sein, wobei zwei Durchführungen bevorzugt um 180° versetzt um den Mittelpunkt von Scheiben des Ventilsitzes und des Ventilkörpers angeordnet sind, wobei die Scheiben die bereichsweise geschlossene Kopfseite des Ventilsitzes und die Dichtfläche des Ventilkörpers bilden.

Ferner kann vorgesehen sein, dass der Ventilkörper den Anschluss zum flüssigkeitsdichten Verbinden einer Knochenzementkartusche aufweist oder mit einem solchen Anschluss fest verbunden ist.

Hierdurch kann der Ventilkörper mit Hilfe einer angeschlossenen Knochenzementkartusche bedient werden.

Gemäß einer bevorzugten Weiterbildung kann vorgesehen sein, dass die Vorrichtung ein Adapterelement aufweist, das mit einer Knochenzementkartusche verbunden ist oder verbindbar ist, wobei das Adapterelement lösbar und formschlüssig mit dem Anschluss verbunden oder verbindbar ist, so dass ein Innenraum der Knochenzementkartusche über das Adapterelement für Knochenzementteig durchlässig mit der mindestens einen zweiten Durchführung im Ventilkörper verbunden oder verbindbar ist.

Hierdurch wird erreicht, dass der Knochenzementteig aus der Knochenzementkartusche problemlos durch das Ventil in seiner geöffneten Stellung hindurch in die Gießform eingefüllt werden kann.

Gemäß einer Weiterbildung der vorliegenden Erfindung kann vorgesehen sein, dass die Vorrichtung eine Knochenzementkartusche zum Mischen von Knochenzement-Ausgangskomponenten und zum Austragen von gemischtem Knochenzementteig aus der Knochenzementkartusche heraus aufweist, bevorzugt eine Knochenzementkartusche zum Mischen von Polymethylmethacrylat-Knochenzement-Ausgangskomponenten und zum Austragen von gemischtem Polymethylmethacrylat-Knochenzementteig aus der Knochenzementkartusche heraus aufweist, wobei besonders bevorzugt die Knochenzementkartusche die Knochenzement-Ausgangskomponenten zur Herstellung des Knochenzements in voneinander getrennten Bereichen beinhaltet.

Hierdurch wird die Vorrichtung weiter komplettiert, da mit der Vorrichtung dann auch der Knochenzementteig bereitgestellt werden kann, der zum Ausformen des Spacers in die Gießform gefüllt wird.

Es kann auch vorgesehen sein, dass die Gießform aus einer Kunststofffolie besteht oder im Wesentlichen aus einer Kunststofffolie besteht oder die Gießform aus zwei oder mehreren Kunststofffolien aufgebaut ist, die miteinander verschweißt oder verklebt sind, und wobei bevorzugt die Gießform aus PETG-Folie und/oder Polyamid-Folie und/oder PE-Folie gefertigt ist.

Hierdurch kann die Gießform und damit die Vorrichtung kostengünstig gefertigt werden. Die genannten Materialien sind zur Formgebung des Spacers aus dem Knochenzementteig geeignet. Zudem lassen sich die Spacer so nach dem Aushärten gut entformen.

Unter dem Begriff "Kunststofffolie" werden flächige Kunststoffe verstanden, die eine Schichtdicke bis zu 2 mm haben. Die Kunststofffolien der Gießformen können durch Kalandrieren oder auch durch Spritzgießen hergestellt sein. Für die Formgebung der Gießformen kommen Tiefziehen und Kunststoffspritzgießen in Betracht. Die Gießformteile könne durch Ultraschallverschweißung, durch Hochfrequenzschweißung, thermischer Verschweißung oder durch Klebung miteinander verbunden sein. Es sind auch Kombinationen der Fügeverfahren möglich. Alternativ können die Teile der Gießform auch mechanisch, zum Beispiel durch Klammern, miteinander verbunden werden. PETG ist ein mit Glykol modifiziertes Polyethylenterephthalat (PET), welches sich durch seine wässrigen Eigenschaften (Viskosität) auszeichnet und für den Spritzguss besonders geeignet ist.

Für die Herstellung der Gießformen können Kunststofffolien aus PETG, Polyamid und Polyethylen (PE) mit einer Schichtdicke von 500 µm bis 2000 µm verwendet werden. Dadurch ist es möglich, Gießformen herzustellen, die einen Druck von 10 N/cm² für eine Zeitraum von wenigen Minuten ohne Aufreißen tolerieren.

Des Weiteren kann vorgesehen sein, dass die Summe aller Öffnungen der mindestens einen ersten Durchführung in der geschlossenen Kopfseite höchstens so groß ist wie die geschlossene Oberfläche der Kopfseite und dass die Summe aller Öffnungen der mindestens einen zweiten Durchführung in der Dichtfläche höchstens so groß ist wie die geschlossene Oberfläche der Dichtfläche.

Hierdurch wird sichergestellt, dass ein stabiles und für den Knochenzementteig undurchlässiges Verschließen des Ventils durch Drehen des Ventilkörpers gegen den Ventilsitz möglich ist.

Ferner kann vorgesehen sein, dass der Ventilsitz ein Innengewinde an der Innenseite aufweist und der Ventilkörper ein passendes Außengewinde an der Außenseite aufweist, so dass der Ventilkörper in den Ventilsitz schraubbar ist.

Durch diese Maßnahme kann eine hohe Dichtwirkung an der Verbindung zwischen dem Ventilkörper und dem Ventilsitz erreichet werden. Zudem kann das Ventil so einfach und kostengünstig zusammengebaut werden.

Auch kann vorgesehen sein, dass der Anschluss zum flüssigkeitsdichten Verbinden einer Knochenzementkartusche ein Innengewinde im Ventilkörper oder ein Außengewinde am Ventilkörper umfasst, wobei vorzugsweise ein Adapterelement der Knochenzementkartusche oder an der Knochenzementkartusche ein zu dem Innengewinde oder zu dem Außengewinde passendes Gegengewinde aufweist.

Hierdurch kann zum Einen eine stabile und flüssigkeitsdichte Verbindung am Anschluss hergestellt werden und zum anderen kann die Drehung bei der Schraubbewegung genutzt werden, um zu Beginn oder nach Ende der Schraubbewegung den Ventilkörper gegen den Ventilsitz zu drehen und somit das Ventil vom geöffneten in den geschlossenen Zustand zu überführen oder um das Ventil vom geschlossenen in den geöffneten Zustand zu überführen.

Durch die Verwendung geeigneter Gewinde kann insbesondere eine zusätzliche Sicherungsfunktion der Vorrichtung dadurch erzielt werden, dass ein Lösen der Knochenzementkartusche nur bei geschlossenem Ventil möglich ist und ein Öffnen des Ventils nur bei angeschlossener Knochenzementkartusche möglich ist.

Es kann vorgesehen sein, dass das Innengewinde im Ventilkörper oder das Außengewinde am Ventilkörper ein Rechtsgewinde ist und das Ventil durch eine gleichsinnige Rechtsdrehung des Ventilkörpers von der geschlossenen in die geöffnete Stellung überführbar ist und das

Ventil durch eine gegensinnige Linksdrehung des Ventilkörpers von der geöffneten in die geschlossene Stellung überführbar ist oder
das Innengewinde im Ventilkörper oder das Außengewinde am Ventilkörper ein Linksgewinde ist und das Ventil durch eine gleichsinnige Linksdrehung des Ventilkörpers von der geschlossenen in die geöffnete Stellung überführbar ist und das Ventil durch eine gegensinnige Rechtsdrehung des Ventilkörpers von der geöffneten in die geschlossene Stellung überführbar ist oder
das Innengewinde des Ventilsitzes und das Innengewinde und das Außengewinde des Ventilkörpers alle Linksgewinde oder alle Rechtsgewinde sind, wobei vorzugsweise auch ein Außengewinde eines Adapterelements zum flüssigkeitsdichten Verbinden einer Knochenzementkartusche an den Anschluss den gleichen Drehsinn hat.

Auch diese Maßnahmen zielen darauf ab, dass ein Schließen des Ventils beim Ausschrauben der Knochenzement-Kartusche automatisch erfolgt und ein Öffnen des Ventils bei Einschrauben der Knochenzement-Kartusche automatisch erfolgt.

Bevorzugt kann auch vorgesehen sein, dass die mindestens eine erste Durchführung in der bereichsweise geschlossenen Kopfseite die gleiche Größe und Gestalt hat wie die mindestens eine zweite Durchführung in der Dichtfläche.

Ebenfalls bevorzugt kann vorgesehen sein, dass die mindestens eine erste Durchführung in der bereichsweise geschlossenen Kopfseite zwei erste Durchführungen sind und die mindestens eine zweite Durchführung in der Dichtfläche zwei zweite Durchführungen sind, wobei bevorzugt die zwei ersten Durchführungen in gegenüberliegend angeordneten Kreissegmentvierteln bezüglich der Drehachse des Ventilkörpers im Ventilsitz angeordnet sind und die zwei zweiten Durchführungen in gegenüberliegend angeordneten Kreissegmentvierteln bezüglich der Drehachse des Ventilkörpers in der Dichtfläche angeordnet sind.

Durch diese beiden Maßnahmen kann ein ausreichender Strömungsquerschnitt für den zähflüssigen Knochenzementteig bereitgestellt werden und es können einseitige Belastungen des Ventils vermieden werden, die ansonsten zu einer Undichtigkeit des Ventils führen könnten.

Des Weiteren kann auch vorgesehen sein, dass an der Dichtfläche des Ventilkörpers ein Bund angeordnet ist, der sich auf einem Rand des Ventilsitzes abstützt oder an der bereichsweise geschlossenen Kopfseite des Ventilsitzes ein Bund angeordnet ist, der sich auf einem Rand des Ventilkörpers abstützt.

Hierdurch kann eine stabile Führung des Ventilkörpers am Ventilsitz erreicht werden. Ferner kann dadurch exakt die Position der mindestens einen zweiten Durchführung bei gegebener Gewindelänge des Ventilkörpers bezüglich der mindestens einen ersten Durchführung definiert werden.

Dabei kann vorgesehen sein, dass ein radial ausgerichteter Hebel an der Mantelfläche des Ventilkörpers neben dem Bund angeordnet ist.

Ferner kann vorgesehen sein, dass an dem Ventilkörper ein Hebel angeordnet ist, der eine radiale Erstreckung bezüglich der Drehachse des Ventilkörpers aufweist, wobei vorzugsweise der Hebel durch eine Aussparung in der Gießform oder in dem Ventilsitz ragt, wobei die Aussparung in der Gießform gegebenenfalls im Bereich der Verbindung zum Ventilsitz angeordnet ist, wobei die Aussparung derart dimensioniert ist, dass sich das Ventil durch Drehen des Ventilkörpers im Ventilsitz mittels des Hebels von der geöffneten Stellung in die geschlossene Stellung und umgekehrt überführen lässt, wobei besonders bevorzugt die Aussparung derart dimensioniert ist, dass sich der Ventilkörper maximal um 90° gegen den Ventilsitz drehen lässt.

Hiermit kann das Ventil bequem von außen manuell bedient werden. Mit diesem Hebel kann der Ventilkörper von der geöffneten Stellung in die geschlossene Stellung des Ventils gedreht werden.

Gemäß einer bevorzugten Weiterbildung kann vorgesehen sein, dass der Ventilkörper und der Ventilsitz aus Kunststoff gefertigt sind, insbesondere aus thermoplastischem Kunststoff gefertigt sind, wobei bevorzugt der Ventilsitz mit einer Wandung der Gießform verklebt oder verschweißt ist.

Hierdurch ist das Ventil und damit die Vorrichtung kostengünstig und als hygienisches Wegwerfprodukt zu fertigen.

Bevorzugt kann vorgesehen sein, dass der Ventilsitz an seiner Außenseite Rippen besitzt, die eine formschlüssige Verbindung mit der Gießform eingehen beziehungsweise eingehen können.

Auch kann vorgesehen sein, dass die Gießform ausgehend von einem Innenraum der Gießform mindestens drei oder vier Kavitäten zur Aufnahme von Haltestiften aufweist, wobei vorzugsweise die Gießform zweiteilig ist und die Kavitäten in Rändern oder in Flanschen zumindest eines Teils der zweiteiligen Gießform angeordnet sind.

Mit diesen Kavitäten kann ein Metallkern als Armierung in dem Spacer angeordnet und genau platziert werden.

Des Weiteren kann vorgesehen sein, dass die Vorrichtung einen Metallkern aufweist, der in der Gießform anzuordnen ist, wobei bevorzugt der Metallkern Bohrungen zur Aufnahme von Haltestiften besitzt, wobei besonders bevorzugt die Bohrungen nicht in einem Bereich der Gießform zum Formen einer Gleitfläche des Spacers angeordnet sind, ganz besonders bevorzugt die Bohrungen in einem Bereich der Gießform zum Formen eines Schafts des Spacers angeordnet sind.

Der Schaft des Spacers dient der Verbindung zum Knochen und wird bei einer Implantation mit Knochenzement abgedeckt.

Vorzugsweise besteht der Metallkern aus einem biokompatiblen Metall oder aus einer biokompatiblen Metalllegierung, besonders bevorzugt aus chirurgischem Stahl.

Es kann auch vorgesehen sein, dass die Vorrichtung zumindest drei oder vier Haltestifte zur Halterung des Metallkerns in der Gießform aufweist.

Der Metallkern dient der Stabilisierung des Spacers und damit einer besseren Verwendbarkeit des behandelten Gelenks.

Durch die Haltestifte wird er Metallkern in einer definierten Position innerhalb der Gießform gehalten. Dadurch wird die Dicke des Knochenzementmantels um den Metallkern definiert. Die Haltestifte sind vorzugsweise aus einem biokompatiblen Kunststoff gefertigt. Besonders eignet sich hierbei Polymethylmethacrylat. Haltestifte aus Polymethylmethacrylat verbinden sich mit dem Knochenzementteig irreversibel. Nach Aushärtung des Knochenzementteigs werden die aus dem Spacer herausragenden Haltestifte einfach abgeschnitten. Die im Inneren des Spacer befindlichen Reste der Haltestifte verbleiben dort.

Auch kann vorgesehen sein, dass in der Gießform zumindest eine Entlüftungsöffnung vorgesehen ist, durch die Luft oder Gas aus dem Inneren der Gießform entweichen kann, wobei bevorzugt wenigstens eine der zumindest eine Entlüftungsöffnung, besonders bevorzugt jede der zumindest einen Entlüftungsöffnung, in einem Bereich der Gießform angeordnet ist, die eine Gleitfläche oder einen Gelenkkopf des Spacers formt.

Hiermit kann Luft oder Gas aus dem Inneren der Gießform entweichen, wenn der Knochenzementteig eingefüllt wird. Dadurch können Lufteinschlüsse und dadurch Unebenheiten an der Oberfläche des Spacers vermieden werden.

Die der vorliegenden Erfindung zugrundeliegenden Aufgaben werden auch gelöst durch ein Verfahren zur Herstellung eines Spacers zum temporären Ersetzen eines Gelenks oder eines Teils eines Gelenks, insbesondere eines Hüftgelenks oder eines Schultergelenks, umfassend eine artikulierende Oberfläche des Gelenks, wobei das Verfahren mit einer erfindungsgemäßen Vorrichtung durchgeführt wird, das Verfahren aufweisend die folgenden chronologischen Schritte:
A) Flüssigkeitsdichtes Verbinden einer Knochenzementkartusche mit dem Anschluss der Vorrichtung;
B) Einpressen von Knochenzementteig aus der Knochenzementkartusche durch das Ventil in der geöffneten Stellung hindurch in die Gießform;
C) Drehen des Ventilkörpers gegen den Ventilsitz und dadurch Überführen des Ventils in die geschlossene Stellung und Abscheren des Knochenzementteigs an der mindestens einen ersten Durchführung in der bereichsweise geschlossenen Kopfseite des Ventilsitzes durch die Drehung des Ventilkörpers gegen den Ventilsitz;
D) Lösen der Knochenzementkartusche von dem Anschluss;
E) Aushärten des Knochenzementteigs in der Gießform; und
F) Entnehmen des so geformten und ausgehärteten Spacers aus der Gießform.

Der Spacer ist für medizinische Anwendungen vorgesehen. Das erfindungsgemäße Verfahren umfasst nicht die Implantation bei einem Patienten sondern lediglich das Ausformen des Spacers. Nach Schritt F) kann der Spacer entgratet, geglättet, geschliffen, gereinigt, poliert und/oder bereichsweise aufgeraut werden.

Zum Entnehmen des geformten und ausgehärteten Spacers aus der Gießform in Schritt F) kann die Gießform nach Schritt E) geöffnet werden.

Bei erfindungsgemäßen Verfahren kann vorgesehen sein, dass nach Schritt D) und vor Schritt E) die folgenden Zwischenschritte erfolgen:
D2) Flüssigkeitsdichtes Verbinden einer neuen Knochenzementkartusche mit dem Anschluss der Vorrichtung, wobei in der neuen Knochenzementkartusche Knochenzementteig oder Ausgangskomponenten zur Herstellung des Knochenzementteigs enthalten ist oder sind;
D3) Drehen des Ventilkörpers gegen den Ventilsitz und dadurch Überführen des Ventils in die geöffnete Stellung;
D4) Einpressen des Knochenzementteigs aus der neuen Knochenzementkartusche durch das Ventil in der geöffneten Stellung hindurch in die Gießform;
D5) Drehen des Ventilkörpers gegen den Ventilsitz und dadurch Überführen des Ventils in die geschlossene Stellung und Abscheren des Knochenzementteigs an der mindestens einen ersten Durchführung in der bereichsweise geschlossenen Kopfseite des Ventilsitzes durch die Drehung des Ventilkörpers gegen den Ventilsitz; und
D6) Lösen der neuen Knochenzementkartusche von dem Anschluss;
wobei vorzugsweise die Schritte D2) bis D6) einmal oder mehrfach mit jeweils neuen Knochenzementkartuschen, die Knochenzementteig oder dessen Ausgangskomponenten enthalten, wiederholt werden, bis die Gießform vollständig oder nach Bedarf mit Knochenzementteig gefüllt ist.

Hierdurch kann eine Gießform mit großem Volumen mit mehreren Knochenzementkartuschen enthaltend geringe Volumina des Knochenzementteigs gefüllt werden. Dies ist beispielsweise für die Herstellung von großvolumigen Hüftgelenkspacern vorteilhaft.

Des Weiteren kann vorgesehen sein, dass vor Schritt B), und vorzugsweise vor Schritt A), der Knochenzementteig in der Knochenzementkartusche aus einer Monomerflüssigkeit und aus einem Zementpulver gemischt wird, wobei bevorzugt gegebenenfalls vor Schritt D3), vorzugsweise vor Schritt D2), der Knochenzementteig in der neuen Knochenzementkartusche aus einer Monomerflüssigkeit und aus einem Zementpulver gemischt wird.

Hierdurch kann zur Herstellung des Spacers ein frisch gemischter Knochenzementteig verwendet werden. Insbesondere PMMA-Knochenzementteige lassen sich im gemischten Zustand nur schwer oder gar nicht über Zeiträume von mehr als wenigen Minuten lagern. Zudem können dem Knochenzementteig so auch kurz vor der Herstellung des Spacers dem Knochenzementteig zur Behandlung geeignete pharmazeutische Wirkstoffe, wie Antibiotika und Antimykotika, beigemengt werden.

Ferner kann vorgesehen sein, dass zum flüssigkeitsdichten Verbinden der Knochenzementkartusche und/oder der neuen Knochenzementkartusche mit dem Anschluss die Knochenzementkartusche oder die neue Knochenzementkartusche in den Anschluss hineingedreht oder eingeschraubt wird und zum Lösen der Knochenzementkartusche und/oder der neuen Knochenzementkartusche von dem Anschluss die Knochenzementkartusche oder die neue Knochenzementkartusche aus dem Anschluss herausgedreht oder herausgeschraubt wird.

Neben dem Schrauben kann die Knochenzementkartusche beispielsweise mit einem Bajonettverschluss mit dem Anschluss verbunden werden.

Durch das Eindrehen oder Einschrauben der Knochenzementkartusche in den Anschluss kann eine flüssigkeitsdichte Verbindung zwischen dem Anschluss und der Knochenzementkartusche bereitgestellt werden. Zudem kann durch die Drehung auch eine Drehung des Ventilkörpers gegen den Ventilsitz erfolgen beziehungsweise induziert werden.

Auch kann vorgesehen sein, dass das Drehen des Ventilkörpers gegen den Ventilsitz durch ein Schrauben des Ventilkörpers in dem Ventilsitz erfolgt oder durch ein manuelles Drehen des Ventilkörpers gegen Ventilsitz erfolgt, wobei bevorzugt das manuelle Drehen durch Bedienen eines sich radial von Ventilkörper weg erstreckenden Hebels erfolgt, der sich durch eine Aussparung in der Gießform oder in dem Ventilsitz erstreckt.

Hiermit kann das Ventil auf einfache Art und Weise vom Anwender bedient werden.

Es kann auch vorgesehen sein, dass das Einpressen des Knochenzementteigs aus der Knochenzementkartusche oder der neuen Knochenzementkartusche durch Eindrücken eines Kolbens in einen Innenraum der Knochenzementkartusche erfolgt.

Hierdurch kann der Knochenzementteig auf einfache Weise aus der Knochenzementkartusche durch das geöffnete Ventil in die Gießform eingepresst werden.

Schließlich kann auch vorgesehen sein, dass vor Schritt B), und vorzugsweise vor Schritt A), ein Metallkern innerhalb der Gießform angeordnet wird, wobei bevorzugt der Metallkern über mehrere Haltestifte von einer Innenwand der Gießform beabstandet wird, wobei besonders bevorzugt die mehreren Haltestifte in Bohrungen im Metallkern und in Kavitäten zur Aufnahme von Haltestiften in der Innenwand der Gießform befestigt werden.

Hierdurch kann der Spacer mit Hilfe der Vorrichtung mit einer innenliegenden Armierung aufgebaut werden. Der Knochenzementteig umfließt dabei den in der Gießform angeordneten Metallkern.

Der Erfindung liegt die überraschende Erkenntnis zugrunde, dass es durch einen in einem Ventilsitz drehbaren Ventilkörper gelingt, eine Vorrichtung mit einer Gießform bereitzustellen, bei der der Anguss mit dem Ventilkörper abgeschert beziehungsweise weitgehend abgeschert werden kann und gleichzeitig bei bestehendem Druck aus einer Knochenzementkartusche die Gießform derart zu verschließen oder die verbleibenden Kanäle zumindest so weit einzuschnüren, dass der Knochenzementteig in der Gießform weiter unter Druck gehalten werden kann, um sich an die Innenseiten der Gießform anzupressen, wobei gleichzeitig verhindert wird, dass Knochenzementteig aus der Gießform durch die Einfüllöffnung wieder herausgedrückt wird. Die Vorrichtung ermöglicht auch, die Gießform mit den Inhalten mehrerer Knochenzementkartuschen nacheinander zu befüllen, ohne dass der Knochenzementteig wieder durch die Einfüllöffnung aus der Gießform herausfließen kann. Hierdurch gelingt es, auch mit Knochenzementkartuschen, die nur geringe Volumina des Knochenzements bereitstellen, Spacer mit großem Volumen herzustellen.

Knochenzementteig, insbesondere nicht hochviskoser Knochenzementteig, kann durch den Verschluss beziehungsweise das geschlossene Ventil nicht aus der Gießform fließen. Eine Ausbildung von Fehlstellen im Spacer infolge des Auslaufens von Knochenzementteig wird dadurch verhindert. Weiterhin wird durch die erfindungsgemäßen Maßnahmen der noch in der Knochenzementkartusche befindliche Rest des Knochenzementteigs vom Knochenzementteig in der Gießform getrennt. Nach beendeter Aushärtung des Knochenzementteigs ist es daher nicht mehr notwendig, den Anguss mechanisch beispielsweise durch Sägen abzutrennen. Eventuell verbliebene dünne Verbindungen können leicht abgebrochen oder abgeschnitten werden. Dadurch wird der Arbeits- und Zeitaufwand für das OP-Personal gesenkt.

Der Anguss des Spacers wird durch die Einfüllöffnung mit dem Ventilsitz und dem Ventilkörper gebildet. Durch das Verdrehen des Ventilkörpers gegenüber dem Ventilsitz aus der geöffneten Stellung in die geschlossene Stellung des Ventils wird die Gießform für Knochenzementteig undurchlässig verschlossen. Das bedeutet, der aus dem Ventilsitz und dem Ventilkörper gebildete Anguss beziehungsweise die dem Anguss formgebende Teile haben gleichzeitig eine Ventilfunktion. Komplexe zusätzliche Ventile sind nicht notwendig.

Die manuelle Drehung des Ventilkörpers gegen den Ventilsitz kann durch einen Hebel an der Außenseite des Ventilkörpers erfolgen. Vorteilhaft kann die Drehung auch dadurch erfolgen, dass beim Herausdrehen der Knochenzementkartusche der Ventilkörper von der Knochenzementkartusche mit gedreht wird. Dabei ist es jedoch notwendig, dass ein Anschlag die Drehbewegung des Ventilkörpers gegenüber dem Ventilsitz begrenzt, damit der Verschluss sicher erfolgen kann und damit der Ventilkörper nicht aus dem Ventilsitz komplett herausgedreht werden kann.

Ein mit der Vorrichtung hergestellter Spacer kann vorteilhaft im Rahmen von zweizeitigen septischen Revisionen verwendet werden, bei denen eine Infektion mit zwei oder mehreren mikrobiellen Keimen und insbesondere mit problematischen Keimen vorliegt.

Eine beispielhafte erfindungsgemäße Vorrichtung kann zusammengesetzt sein aus
a) einer Knochenzementkartusche zum Mischen von Polymethylmethacrylat-Knochenzement-Komponenten und zum Austragen von gemischtem Polymethylmethacrylat-Knochenzementteig,
b) einem Adapterelement, das mit dem Kartuschenkopf der Knochenzementkartusche verbunden ist oder verbindbar ist, wobei das Adapterelement eine reversible formschlüssige Verbindung ausbilden kann und wobei das Adapterelement eine Durchführung besitzt, die den Innenraum der Zementkartusche flüssigkeitsdurchlässig mit der Umgebung verbindet,
c) einer aus Kunststofffolie gefertigten hohlen Gießform, die eine Einfüllöffnung zum Eintrag des Knochenzementteigs besitzt,
d) einem formstabilen hohlzylinderförmigen Ventilsitz,
   d1) der in der Einfüllöffnung der Gießform zum Eintrag des Knochenzementteigs verdrehsicher angeordnet ist,
   d2) wobei der formstabile Ventilsitz flüssigkeitsundurchlässig mit der Gießformwand verbunden ist,
   d3) wobei der formstabile Ventilsitz an der Stirnseite, die den Hohlraum der Gießform begrenzt, als Scheibe ausgebildet ist, die mindestens eine erste Durchführung besitzt, die den Innenraum der Gießform mit der Umgebung flüssigkeitsdurchlässig verbindet, wobei die Fläche der mindestens einen ersten Durchführung nur maximal 50 Prozent der Scheibenfläche der Scheibe des Ventilsitzes einnimmt, und
   d4) wobei der formstabile Ventilsitz ein Innengewinde an der Innenseite besitzt,
e) einen formstabilen, hohlzylinderförmigen Ventilkörper,
   e1) welcher ein Außengewinde an der Außenseite des Hohlzylinders besitzt, das mit dem Innengewinde des formstabilen Ventilsitzes verschraubbar ist,
   e2) wobei ein Innengewinde oder Teile eines Innengewindes an einer Innenseite des Hohlzylinders des Ventilkörpers angeordnet sind, welche mit dem Adapterelement reversibel durch Verschrauben verbindbar ist oder sind,
   e3) wobei der Ventilkörper an einer Stirnseite, die dem Hohlraum der Gießform zugewandt ist, als Scheibe ausgebildet ist, die mindestens eine zweite Durchführung besitzt, wobei die Fläche der mindestens einen zweiten Durchführung nur maximal 50 Prozent der Scheibenfläche der Scheibe des Ventilkörpers einnimmt, und
   e4) wobei die mindestens eine zweite Durchführung eine annähernd gleiche Größe und Gestalt wie die mindestens eine erste Durchführung des formstabilen Ventilsitzes hat, und
f) wobei der Ventilkörper nach Einschrauben in den formstabilen Ventilsitz in einer geöffneten Stellung so gedreht wird, dass die mindestens eine zweite Durchführung über der mindestens einen ersten Durchführung liegt und eine flüssigkeitsdurchlässige Verbindung vom Innenraum der Gießform mit der Umgebung beziehungsweise der Knochenzementkartusche hergestellt wird und in eine geschlossene Stellung gedreht werden kann, so dass die mindestens eine zweite Durchführung nicht die mindestens eine erste Durchführung überdeckt.

Nach Beendigung des Einpressens des Knochenzementteigs in den Hohlraum der Gießform wird der Ventilkörper manuell so gedreht, dass die mindestens eine zweite Durchführung des Ventilkörpers nicht mehr über der mindestens einen ersten Durchführung des formstabilen Ventilsitzes steht beziehungsweise fluchtet. Dadurch wird der Hohlraum der Gießform flüssigkeitsundurchlässig verschlossen. Auch ein nicht hochviskoser Knochenzementteig kann dadurch nicht aus der Gießform fließen.

Ein beispielhaftes erfindungsgemäßes Verfahren zur Herstellung von Spacern mit der erfindungsgemäßen Vorrichtung kann die folgenden nacheinander ablaufenden Schritte umfassen:
a) Bereitstellen der Gießform,
b) Mischen des Knochenzementpulvers mit der Monomerflüssigkeit in einer Knochenzementkartusche bis sich ein Knochenzementteig gebildet hat, wobei die Knochenzementkartusche ein Adapterelement am Kartuschenkopf besitzt, das flüssigkeitsdurchlässig mit dem Innenraum der Kartusche verbunden ist,
c) Flüssigkeitsdurchlässiges Verbinden der Knochenzementkartusche mit der Gießform durch Einschrauben des Adapterelements in ein Innengewinde des Ventilkörpers,
d) Einpressen des Knochenzementteigs aus der Knochenzementkartusche in die Gießform mit Hilfe einer Auspressvorrichtung, wobei der Knochenzementteig die Luft im Hohlraum der Gießform verdrängt und vorzugsweise die verdrängte Luft durch mindestens eine Entlüftungsöffnung aus dem Hohlraum der Gießform in die Umgebung austritt,
e) Herausdrehen der Knochenzementkartusche mit dem Adapterelement aus dem Ventilkörper, wobei der Ventilkörper aus der geöffneten Stellung in die geschlossene Stellung des Ventils gedreht wird, wodurch die mindestens eine erste Durchführung und die mindestens eine zweite Durchführung nicht mehr übereinanderstehen, so dass kein Knochenzementteig aus dem Hohlraum der Gießform durch das Ventil in die Umgebung austreten kann,
f) Trennen der Knochenzementkartusche von der Gießform
g) Aushärten des Knochenzementteigs in der Gießform,
h) Öffnen der Gießform nach erfolgter Aushärtung des Knochenzementteigs und
i) Entnahme des Spacers.

Ein alternatives beispielhaftes erfindungsgemäßes Verfahren zur Herstellung von Spacern mit der erfindungsgemäßen Vorrichtung kann die folgenden nacheinander ablaufenden Schritte umfassen:
a) Bereitstellen der Gießform,
b) Mischen des Knochenzementpulvers mit der Monomerflüssigkeit in einer Knochenzementkartusche bis sich ein Knochenzementteig gebildet hat, wobei die Knochenzementkartusche ein Adapterelement am Kartuschenkopf besitzt, das flüssigkeitsdurchlässig mit dem Innenraum der Kartusche verbunden ist,
c) Flüssigkeitsdurchlässiges Verbinden der Knochenzementkartusche mit der Gießform durch Einschrauben des Adapterelements in ein Innengewinde des Ventilkörpers,
d) Einpressen des Knochenzementteigs aus der Knochenzementkartusche in die Gießform mit Hilfe einer Auspressvorrichtung, wobei der Knochenzementteig die Luft im Hohlraum der Gießform verdrängt und vorzugsweise die verdrängte Luft durch mindestens eine Entlüftungsöffnung aus dem Hohlraum der Gießform in die Umgebung austritt,
e) Manuelles Drehen eines Hebels am Ventilkörper gegen die Drehrichtung des Gewindes des Ventilkörpers, wobei der Ventilkörper aus der geöffneten Stellung in die geschlossene Stellung des Ventils gedreht wird, wodurch die mindestens eine erste Durchführung und die mindestens eine zweite Durchführung nicht mehr übereinanderstehen, so dass kein Knochenzementteig aus dem Hohlraum der Gießform durch das Ventil in die Umgebung austreten kann,
f) Herausdrehen der Knochenzementkartusche mit dem Adapterelement aus dem Ventilkörper,
g) Trennen der Knochenzementkartusche von der Gießform
h) Aushärten des Knochenzementteigs in der Gießform,
i) Öffnen der Gießform nach erfolgter Aushärtung des Knochenzementteigs und
j) Entnahme des Spacers.

Im Folgenden werden weitere Ausführungsbeispiele der Erfindung anhand von achtundzwanzig schematisch dargestellten Figuren erläutert, ohne jedoch dabei die Erfindung zu beschränken. Dabei zeigt:
Figur 1: eine schematische perspektivische Querschnittansicht einer beispielhaften ersten erfindungsgemäßen Vorrichtung zum Herstellen eines Hüftgelenkspacers mit offenem Ventil;
Figur 2: eine schematische perspektivische Außenansicht der ersten erfindungsgemäßen Vorrichtung nach Figur 1;
Figur 3: eine schematische perspektivische Querschnittansicht der ersten erfindungsgemäßen Vorrichtung nach den Figuren 1 und 2 mit offenem Ventil;
Figur 4: eine schematische Querschnittansicht der ersten erfindungsgemäßen Vorrichtung mit offenem Ventil vor dem Einfüllen von Knochenzementteig in eine Gießform der Vorrichtung;
Figur 5: eine schematische Querschnittansicht der ersten erfindungsgemäßen Vorrichtung während dem Einfüllen von Knochenzementteig in die Gießform;
Figur 6: eine schematische Querschnittansicht der ersten erfindungsgemäßen Vorrichtung mit einer mit Knochenzementteig gefüllten Gießform;
Figur 7: eine schematische Querschnittansicht der ersten erfindungsgemäßen Vorrichtung mit geschlossenem Ventil nach dem Entfernen einer Knochenzementkartusche und eines Adapterelements von der Gießform;
Figur 8: eine schematische perspektivische Ansicht auf die geöffnete erste erfindungsgemäße Vorrichtung mit offenem Ventil ohne den zweiten Teil der Gießform mit eingelegtem Metallkern;
Figur 9: eine schematische perspektivische Außenansicht auf eine Seite mit dem Anschluss der ersten erfindungsgemäßen Vorrichtung mit offenem Ventil;
Figur 10: eine schematische perspektivische Ansicht auf einen Metallkern für eine erfindungsgemäße Vorrichtung nach den Figuren 1 bis 9;
Figur 11: eine schematische perspektivische Querschnittansicht einer beispielhaften zweiten erfindungsgemäßen Vorrichtung zum Herstellen eines Schultergelenkspacers mit offenem Ventil;
Figur 12: eine schematische perspektivische Ansicht auf die geöffnete zweite erfindungsgemäße Vorrichtung nach Figur 11 ohne Metallkern;
Figur 13: eine schematische perspektivische Ansicht auf die geöffnete zweite erfindungsgemäße Vorrichtung ohne den zweiten Teil der Gießform mit eingelegtem Metallkern;
Figur 14: eine schematische perspektivische Außenansicht auf die geschlossene zweite erfindungsgemäßen Vorrichtung nach den Figuren 11 bis 13 mit offenem Ventil;
Figur 15: eine schematische perspektivische Außenansicht auf die geschlossene zweite erfindungsgemäßen Vorrichtung nach Figur 14 mit angeschlossener Knochenzementkartusche;
Figur 16: eine schematische Querschnittansicht der zweiten erfindungsgemäßen Vorrichtung mit offenem Ventil nach dem Einfüllen von Knochenzementteig aus der Knochenzementkartusche in eine Gießform der Vorrichtung;
Figur 17: eine schematische Querschnittansicht der zweiten erfindungsgemäßen Vorrichtung mit geschlossenem Ventil nach dem Entfernen einer Knochenzementkartusche und eines Adapterelements von der Gießform;
Figur 18: eine schematische perspektivische Außenansicht auf die geschlossene zweite erfindungsgemäßen Vorrichtung nach den Figuren 11 bis 17 mit geschlossenem Ventil;
Figur 19: eine schematische perspektivische Querschnittansicht der zweiten erfindungsgemäßen Vorrichtung mit geschlossenem Ventil nach dem Einfüllen von Knochenzementteig;
Figur 20: eine perspektivische Ansicht auf einen Spacer, der mit einer zweiten erfindungsgemäßen Vorrichtung nach den Figuren 11 bis 19 hergestellt wurde;
Figur 21: eine Seitenansicht auf den Spacer nach Figur 20, bei dem vorspringende Haltestifte entfernt wurden;
Figur 22: eine schematische perspektivische Ansicht auf ein Ventil für eine erfindungsgemäße Vorrichtung im geöffneten Zustand;
Figur 23: eine schematische perspektivische Teilquerschnittansicht auf das Ventil nach Figur 22 im geöffneten Zustand;
Figur 24: eine schematische perspektivische Querschnittansicht durch das Ventil nach den

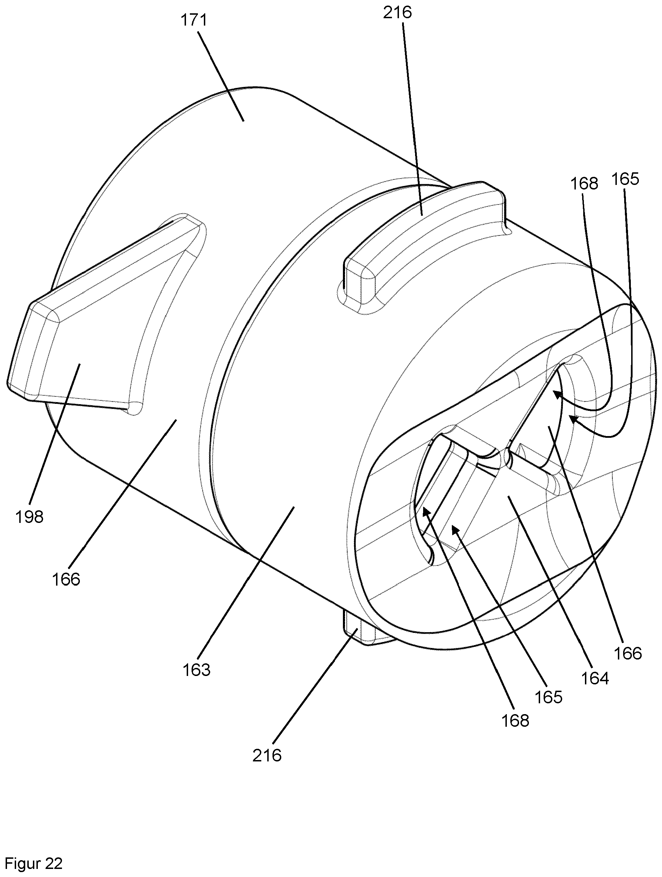

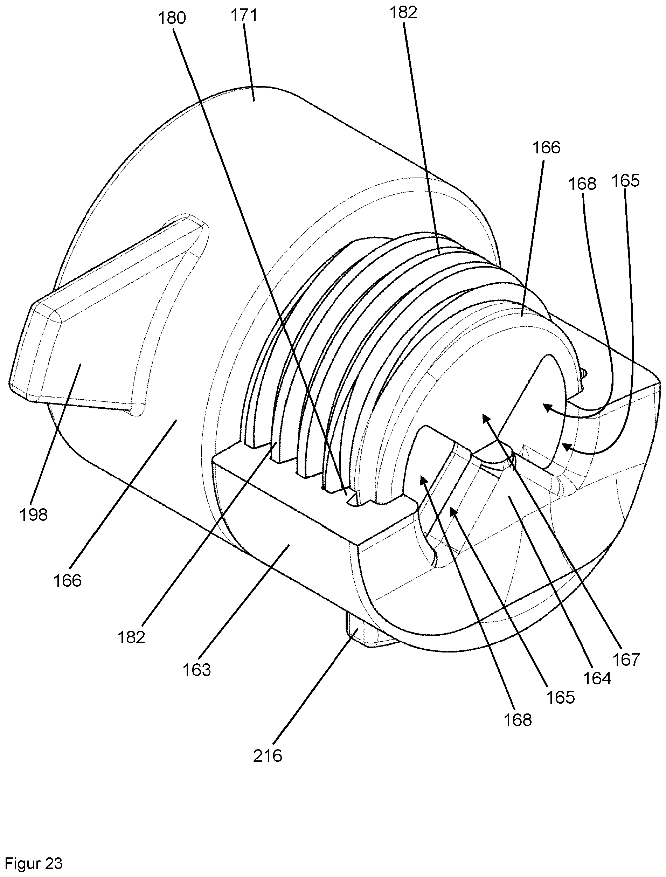

Figuren 22 und 23 im geöffneten Zustand;

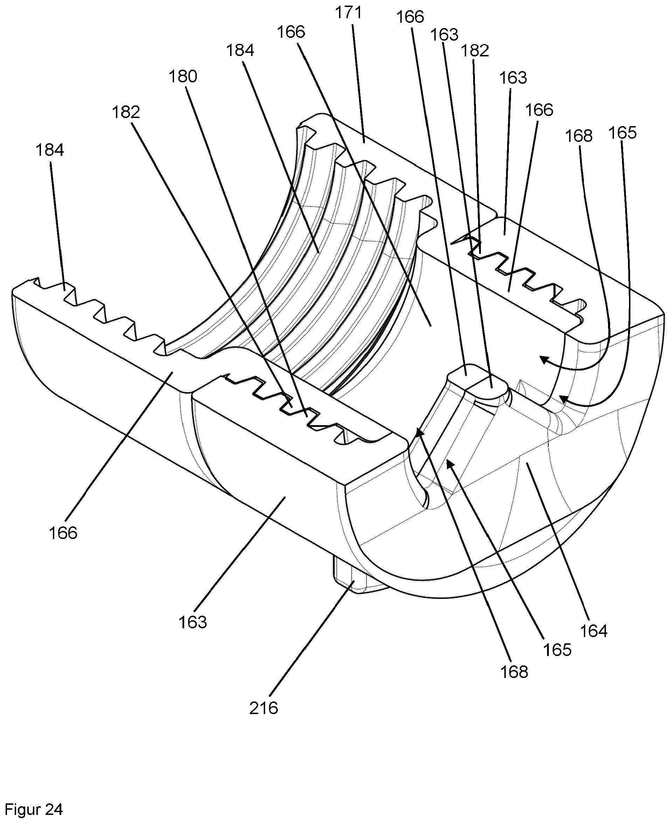

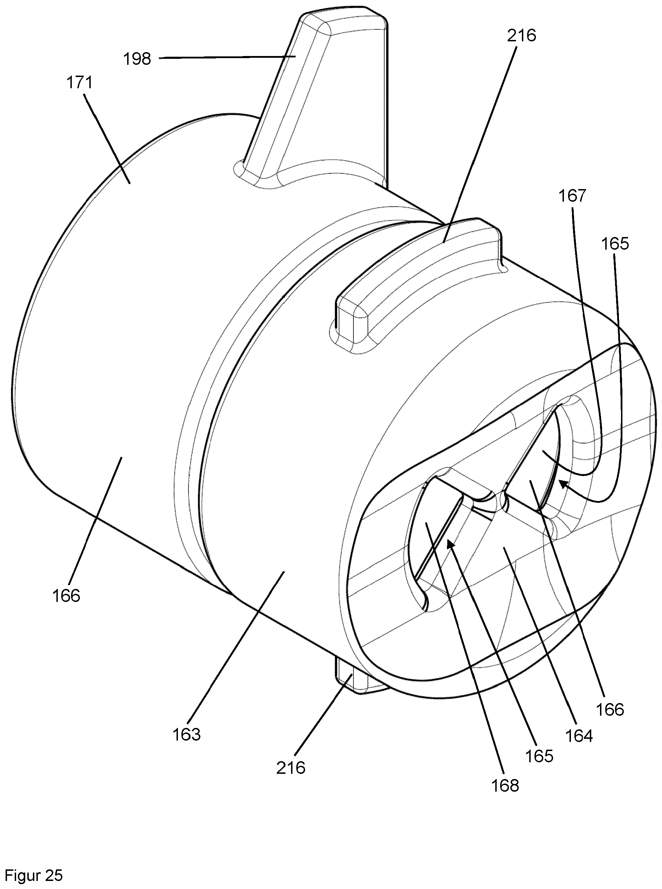

Figur 25: eine schematische perspektivische Ansicht auf das Ventil nach den Figuren 22 bis 24 im geschlossenen Zustand;

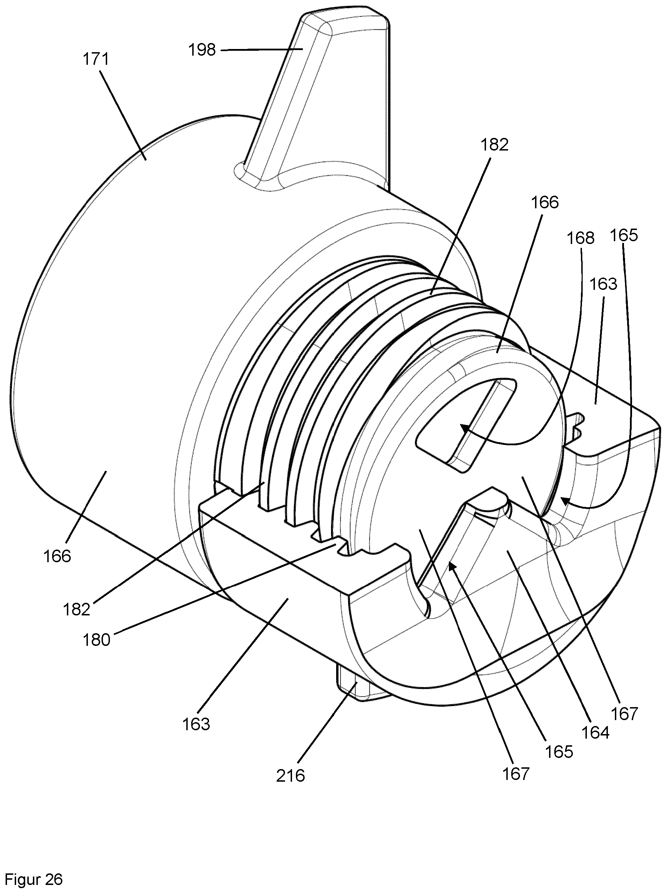

Figur 26: eine schematische perspektivische Teilquerschnittansicht auf das Ventil nach den
Figuren 22 bis 25 im geschlossenen Zustand;

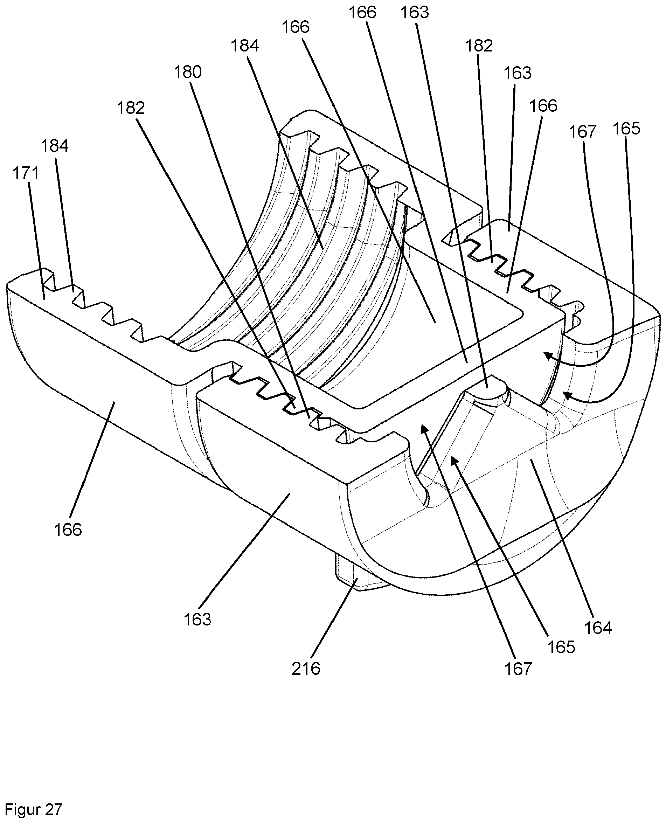

Figur 27: eine schematische perspektivische Querschnittansicht auf das Ventil nach den
Figuren 22 bis 26 im geschlossenen Zustand; und

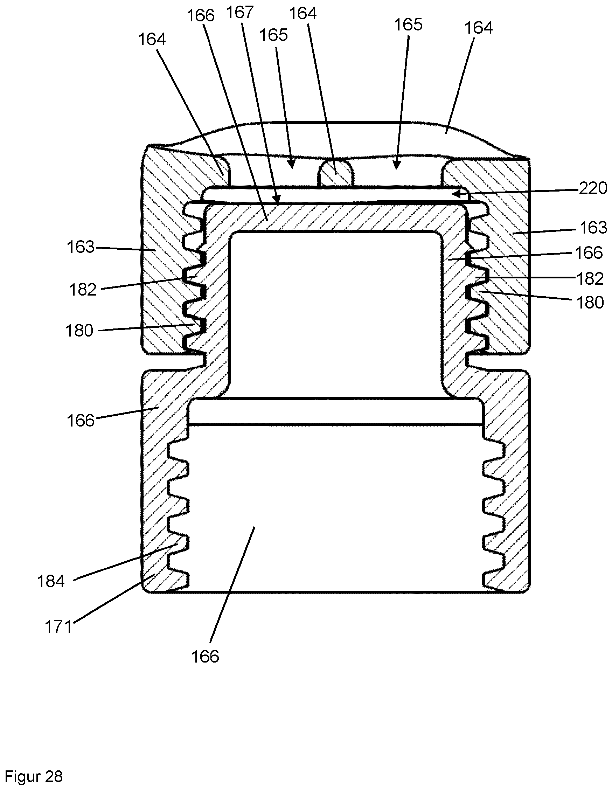

Figur 28: eine schematische Querschnittansicht des Ventils nach den Figuren 22 bis 27 im geschlossenen Zustand.

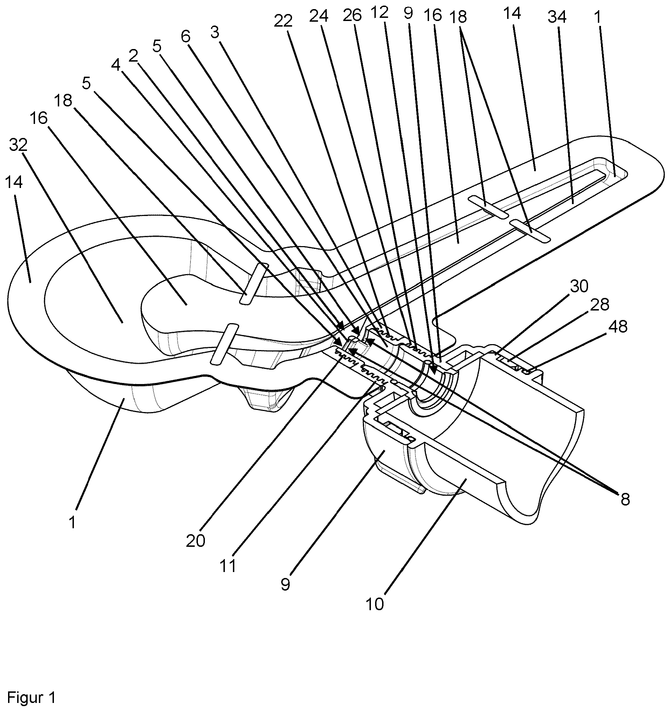

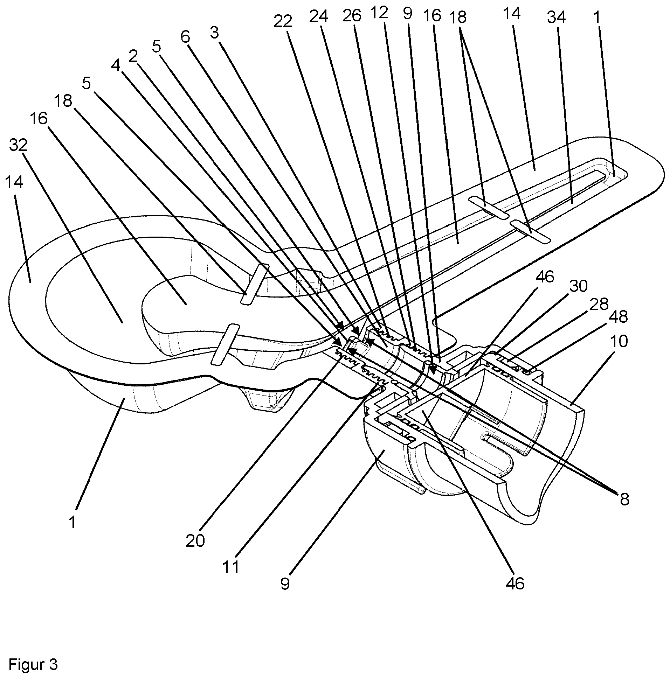

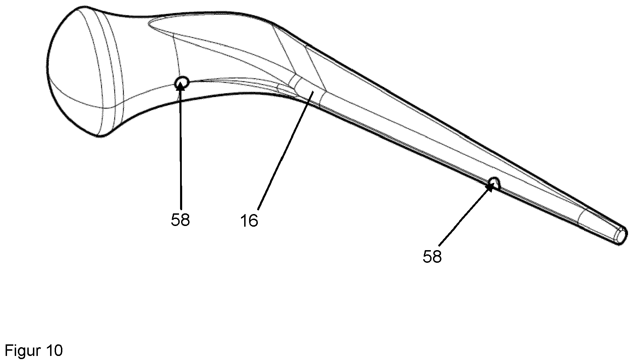

In den Figuren 1 bis 10 sind Abbildungen eines ersten Ausführungsbeispiels einer erfindungsgemäßen Vorrichtung zur Herstellung eines Hüftgelenkspacers und Teile davon in verschiedenen Ansichten gezeigt.

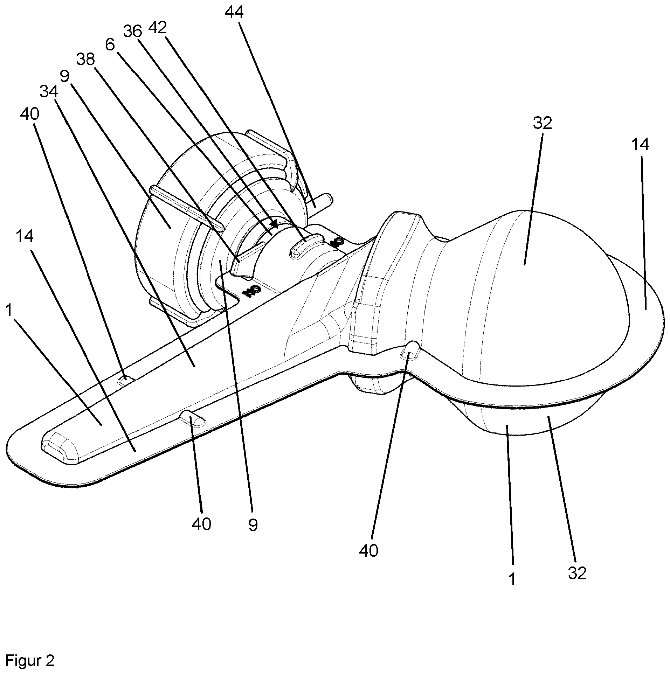

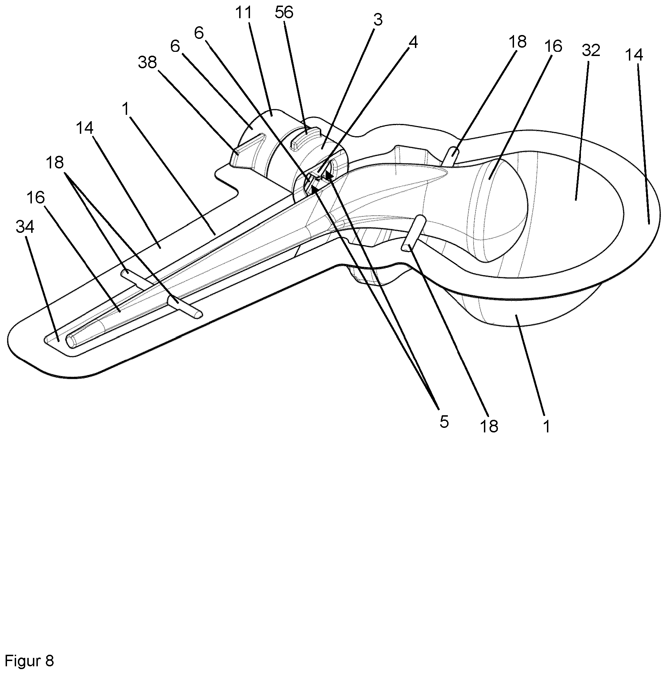

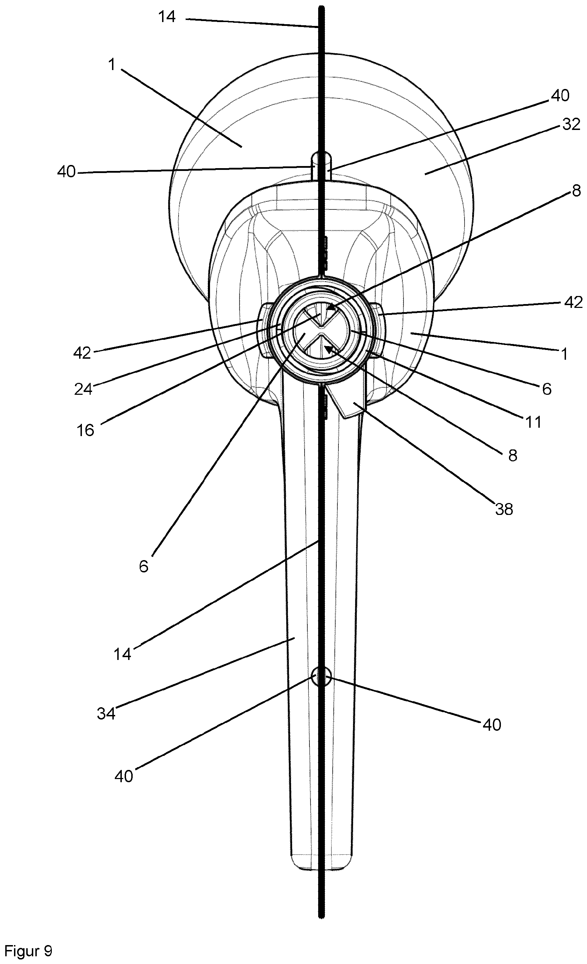

Die erste erfindungsgemäße Vorrichtung ist zur Herstellung eines Spacers für ein Hüftgelenk geeignet und vorgesehen. Die Vorrichtung umfasst eine Gießform 1. Die Gießform 1 kann zweiteilig aufgebaut sein. In den Figuren 1 und 3 bis 8 ist jeweils nur einer der beiden Teile der Gießform 1 erkennbar. In den Figuren 2 und 9 sind beiden Teile der Gießform 1 dargestellt. An einer Seite der Gießform 1 kann eine Einfüllöffnung 2 zum Einfüllen von Knochenzementteig 50 ausgeformt sein, die durch eine in beiden Teilen der Gießform 1 jeweils Halbkreisförmige zylindrische Öffnung definiert sein kann. In dieser Einfüllöffnung 2 kann ein Ventilsitz 3 angeordnet sein. Der Ventilsitz 3 kann mit einem Teil der Gießform 1 fest verbunden sein, so wie das in Figur 8 dargestellt ist. Zur besseren und dichteren Verbindung des Ventilsitzes 3 an die Gießform 1, kann der Ventilsitz 3 an seiner äußeren Oberfläche eine Strukturierung aufweisen, beispielsweise Längsrillen, die parallel zur Zylinderachse einer zylindrischen Außenwand des Ventilsitzes 3 angeordnet sind.

Der Ventilsitz 3 kann als ein Hohlzylinder ausgeführt sein, der auf einer in Richtung der Einfüllöffnung 2 ausgerichteten Kopfseite 4 bis auf zwei erste Durchführungen 5 geschlossen ist. Die beiden ersten Durchführungen 5 können viertelkreisförmig geformt sein und können vorzugsweise um 180° bezüglich der Zylinderachse des Ventilsitzes 3 gegeneinander verdreht beziehungsweise versetzt angeordnet sein. Im Inneren des Ventilsitzes 3 kann ein gegen den Ventilsitz 3 axial verdrehbarer Ventilkörper 6 angeordnet sein. Der Ventilkörper 6 kann eine in Richtung der Kopfseite 4 des Ventilsitzes 3 ausgerichtete Dichtfläche 7 beziehungsweise Oberfläche aufweisen. Der Ventilkörper 6 kann als abgestufter Hohlzylinder aufgebaut sein, dessen vorderer Teil in den Ventilsitz 3 eingeschraubt oder eingesteckt sein kann.

In der Dichtfläche 7 können zwei zweite Durchführungen 8 angeordnet sein. Die beiden zweiten Durchführungen 8 können analog den ersten Durchführungen 5 viertelkreisförmig geformt sein und können vorzugsweise um 180° bezüglich der Zylinderachse des Ventilkörpers 6 gegeneinander verdreht angeordnet sein. Der Ventilsitz 3 und der Ventilkörper 6 bilden zusammen ein Ventil der Vorrichtung. In den Ventilkörper 6 kann ein Adapterelement 9 zum flüssigkeitsdichten Verbinden einer Knochenzementkartusche 10 eingeschraubt sein. Die Knochenzementkartusche 10 und das Adapterelement 9 können Teil der erfindungsgemäßen Vorrichtung sein. Der Ventilkörper 6 kann an seiner offenen Seite, die von der Dichtfläche 7 abgewandt ist, als Anschluss 11 zum Verbinden des Adapterelements 9 ausgeformt sein.

Die Knochenzementkartusche 10 kann an ihrer Vorderseite eine Austragsöffnung 12 zum Austragen des Knochenzementteigs 50 aus der Knochenzementkartusche 10 aufweisen. Die Austragsöffnung 12 kann in dem Adapterelement 9 angeordnet sein und durch dieses begrenzt werden. Das Adapterelement 9 kann die Knochenzementkartusche 10 an ihrer Vorderseite bis auf die Austragsöffnung 12 und gegebenenfalls bis auf einen Vakuumanschluss 44 verschließen.

Die Gießform 1 kann kostengünstig aus Kunststofffolie gefertigt werden. Die Kunststofffolie kann mehrere Schichten aufweisen. Die beiden Teile der Gießform 1 können über Flansche 14 aneinander befestigt werden. Durch Verbinden der beiden Teile der Gießform 1 über die Flansche 14 kann die Gießform 1 nach außen verschlossen werden. In der Gießform 1 können Entlüftungsöffnungen (in den Figuren 1 bis 9 nicht zu sehen) angeordnet sein. Durch die Entlüftungsöffnungen kann Luft oder Gas aus dem Inneren der geschlossenen Gießform 1 entweichen, wenn ein Knochenzementteig 50 durch die Einfüllöffnung 2 in die Gießform 1 eingefüllt wird.

Im Inneren der Gießform 1 kann ein Metallkern 16 platziert werden. Der Metallkern 16 kann aus chirurgischem Stahl oder aus Titan bestehen. Alternativ wäre es theoretisch auch möglich, den Metallkern 16 aus einem Kunststoff wie PMMA zu fertigen. Der Metallkern 16 kann über Haltestifte 18 mit der Gießform 1 verbunden werden. Der Metallkern 16 kann mit Hilfe der Haltestifte 18 von der Innenwand der Gießform 1 beabstandet sein, so dass der Knochenzementteig 50 den Metallkern 16 vollständig umfließen kann. Der Metallkern 16 bewirkt eine Stabilisierung des Spacers. Die Haltestifte 18 können aus PMMA bestehen. Dieses kann sich mit einem Knochenzementteig 50 aus PMMA irreversibel verbinden.

Der Ventilsitz 3 kann an seiner Innenseite ein Innengewinde 20 aufweisen. An der der Dichtfläche 7 zugewandten vorderen Hälfte des Ventilkörpers 6 kann der Ventilkörper 6 an seiner Außenseite ein zum Innengewinde 20 des Ventilsitzes 3 passendes Außengewinde 22 aufweisen. Der Ventilkörper 6 kann mit seinem Außengewinde 22 in das Innengewinde 20 des Ventilsitzes 3 eingeschraubt werden.

Durch Einschrauben des Ventilkörpers 6 in den Ventilsitz 3 bis zum Anschlag können die ersten Durchführungen 5 und die zweiten Durchführungen 8 in Überdeckung zueinander gebracht werden. Das Ventil befindet sich dann im geöffneten Zustand. Ein Knochenzementteig 50 kann in diesem geöffneten Zustand durch die ersten Durchführungen 5 und durch die zweiten Durchführungen 8 aus der Knochenzementkartusche 10 in die Gießform 1 fließen. Durch eine Vierteldrehung (um 90°) des Ventilkörpers 6 gegen den Ventilsitz 3, das heißt durch ein Herausschrauben des Ventilkörpers 6 aus dem Ventilsitz 3, können die ersten Durchführungen 5 und die zweiten Durchführungen 8 gegeneinander versetzt werden, so dass die Dichtfläche 7 des Ventilkörpers 6 die ersten Durchführungen 5 des Ventilsitzes 3 abdeckt und die geschlossenen Bereiche der Kopfseite 4 des Ventilsitzes 3 die zweiten Durchführungen 8 des Ventilkörpers 6 abdeckt. Das Ventil befindet sich dann im geschlossenen Zustand. Durch den geringen Hub des Ventilkörpers 6 gegen den Ventilsitz 3 bei einer Vierteldrehung, ist der zwischen dem Ventilkörper 6 und dem Ventilsitz 3 entstehende Spalt so schmal (weniger als 1 mm breit), dass ein normaler und erst recht ein hochviskoser Knochenzementteig 50 nicht durch den Spalt dringen können. Dies gilt insbesondere, weil der Knochenzementteig 50 im Spalt von seiner eigentlichen Flussrichtung um 90° abgelenkt wird.

In der Rückseite des Ventilkörpers 6 kann im Anschluss 11 ein Innengewinde 24 angeordnet sein. Das Adapterelement 9 weist an seiner Vorderseite ein zum Innengewinde 24 passendes Außengewinde 26 auf. Das Adapterelement 9 kann so in den Anschluss 11 des Ventilkörpers 6 eingeschraubt werden. Hierdurch kann eine flüssigkeitsdichte Verbindung der Knochenzementkartusche 10 zum Ventilkörper 6 und damit in die Gießform 1 erzeugt werden. Das Innengewinde 20 des Ventilsitzes 3, das Außengewinde 22 des Ventilkörpers 6, das Innengewinde 24 des Ventilkörpers 6 und das Außengewinde 26 des Adapterelements 9 können alle den gleichen Drehsinn aufweisen, das heißt, dass alle diese Gewinde Rechtsgewinde oder Linksgewinde sind. Dadurch kann das Ventil durch Einschrauben des Adapterelements 9 in den Anschluss 11 und gleichsinniges Weiterdrehen des Adapterelements 9 geöffnet werden. Gleichzeitig dichtet auch der Ventilkörper 6 gegen den Ventilsitz 3 ab.

Das Adapterelement 9 kann über ein Rastmittel 28 am Adapterelement 9 mit einer Gegenrastung 30 an einer zylindrischen Wandung der Knochenzementkartusche 10 verbunden werden oder sein. Zur Abdichtung kann eine umlaufende Dichtung 48 vorgesehen sein, die die zylindrische Wandung der Knochenzementkartusche 10 gegen das Adapterelement 9 abdichtet.

Die Gießform 1 kann einen Gelenkkopf-Formteil 32 zum Ausformen eines Gelenkkopfs eines Spacers und einen Schaft-Formteil 34 zum Ausformen eines Schafts eines Spacers aufweisen. Des Weiteren kann in der Gießform 1 im Bereich der Einfüllöffnung 2 eine Aussparung 36 für einen Hebel 38 des Ventilkörpers 6 angeordnet sein. Der Hebel 38 kann mit dem Ventilkörper 6 verbunden sein. Der Ventilkörper 6 kann mit dem Hebel 38 im Ventilsitz 3 gedreht werden. Die Aussparung 36 ist vorzugsweise genau so groß, dass den Ventilkörper 6 nur um maximal eine Vierteldrehung gegen den Ventilsitz 3 gedreht werden kann. Dadurch kann das Ventil mit Hilfe des Hebels 38 manuell von außen vom geöffneten Zustand in den geschlossenen Zustand beziehungsweise vom geschlossenen Zustand in den geöffneten Zustand überführt werden.

Im Bereich der Flansche 14 können in der Gießform Formen 40 für Kavitäten angeordnet sein, in denen die Haltestifte 18 angeordnet werden können. Ferner können in der Gießform 1 in den die Einfüllöffnung 2 begrenzenden Wandungen Formen 42 zur Ventilbefestigung angeordnet sein, die zur Aufnahme passender Vorsprünge 56 (siehe Figur 8) an der zylindrischen Außenseite des Ventilsitzes 3 vorgesehen sind. Durch Eingreifen der Vorsprünge 56 in die Formen 42 zur Ventilbefestigung kann der Ventilsitz 3 nicht in der Einfüllöffnung 2 gedreht werden und es wird eine stabile Verbindung bereitgestellt.

In dem Adapterelement 9 kann ein Vakuumanschluss 44 angeordnet sein, mit dem ein Innenraum der Knochenzementkartusche 10, in dem der Knochenzementteig 50 gemischt wird, evakuiert werden kann. Dadurch kann der Knochenzementteig 50 unter Vakuum gemischt werden.

Im zylindrischen Innenraum der Knochenzementkartusche 10 kann ein Kolben 46 zum Austreiben des Knochenzementteigs 50 aus der Knochenzementkartusche 10 durch das Ventil in die Gießform 1 angeordnet sein. Hierzu kann der Kolben 46 an seiner Außenseite zylindrisch geformt und über zwei umlaufende Dichtungen 54 gegen den zylindrischen Innenraum abgedichtet sein. Durch Vortreiben des Kolbens 46 kann der Knochenzementteig 50 aus der Austragsöffnung 12 der Knochenzementkartusche 10 in beziehungsweise durch das geöffnete Ventil gepresst werden.

In dem Adapterelement 9 kann eine Porenscheibe 52 angeordnet sein. Die Porenscheibe 52 ist für den Knochenzementteig 50 und seine Ausgangskomponenten undurchlässig. Der Vakuumanschluss 44 kann von der Porenscheibe 52 abgedeckt sein. Hierdurch wird verhindert, dass ein Knochenzementpulver als Ausgangskomponente des Knochenzementteigs 50 in den Vakuumanschluss 44 eindringen kann.

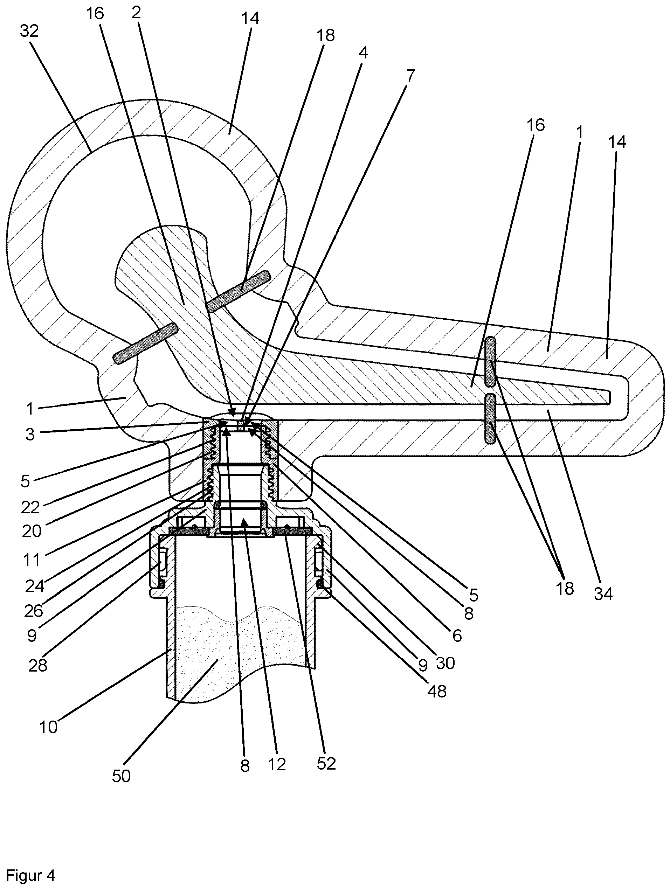

Der Ablauf eines erfindungsgemäßen Verfahrens ist in den Figuren 4 bis 7 anhand der ersten erfindungsgemäßen Vorrichtung dargestellt. Zunächst kann der Metallkern 16 mit den Haltestiften 18 in der Gießform 1 positioniert werden. Hierzu können die Haltestifte 18 an einem Ende zwischen den beiden Teilen der Gießform 1 in den durch die Formen 40 gebildeten Kavitäten angeordnet und gehalten und mit dem anderen Ende in passende Bohrungen 58 im Metallkern 16 angeordnet werden. Die Gießform 1 kann dann geschlossen werden.

Ein Knochenzementteig 50 kann in der Knochenzementkartusche 10 unter Vakuum gemischt werden. Anschließend kann die Knochenzementkartusche 10 mit dem Adapterelement 9 in den Anschluss 11 des Ventilkörpers 6 geschraubt werden. Beim Einschrauben der Adapterelements 9 kann das Ventil in die geöffnete Stellung überführt werden, indem der Ventilkörper 6 bis zum Anschlag in den Ventilsitz 3 geschraubt wird. Diese Situation ist in Figur 4 dargestellt.

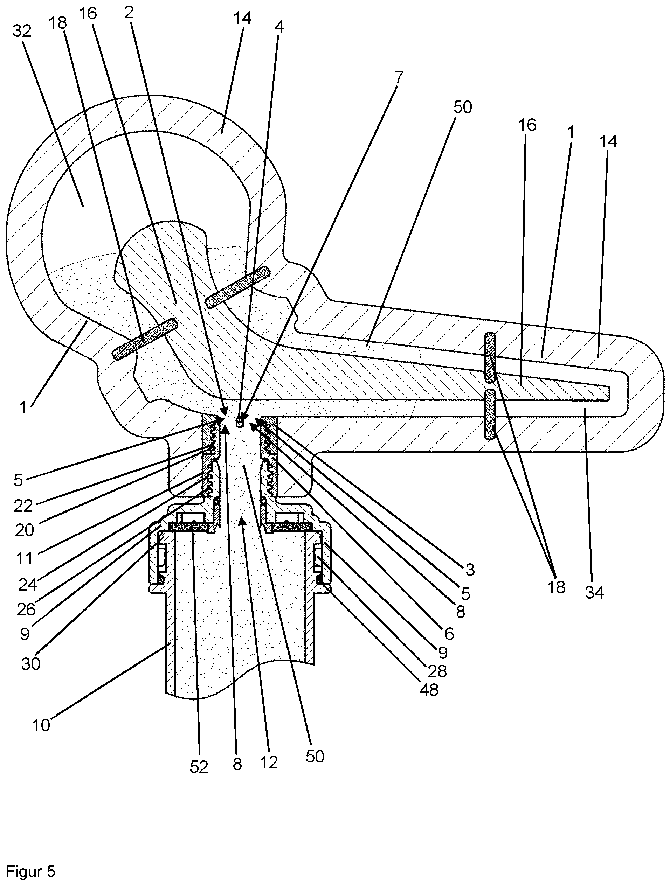

Anschließend wird durch Vortreiben des Kolbens 46 der Knochenzementteig 50 aus der Knochenzementkartusche 10 durch das Ventil und durch die sich in Deckung befindlichen ersten Durchführungen 5 und zweiten Durchführungen 8 in die Gießform 1 gepresst. Diese Situation ist in Figur 5 gezeigt. Durch Schließen des Ventils durch manuelles Bedienen des Hebels 38 und dadurch Drehen des Ventilkörpers 6 um eine Vierteldrehung gegen den Ventilsitz 3 kann zwischendurch eine neue Knochenzementkartusche 10 angesetzt werden, falls das Volumen des Knochenzementteigs 50 einer einzigen Knochenzementkartusche 10 nicht ausreicht, um die Gießform 1 vollständig zu füllen. Dabei kann der in der Gießform 1 enthaltene Knochenzementteig 50 nicht wieder ausfließen, das die ersten Durchgänge 5 und die zweiten Durchgänge 8 in der geschlossenen Stellung des Ventils abgedeckt sind und der Spalt dazwischen nicht ausreicht, um den viskosen Knochenzementteig 50 hindurchfließen lassen zu können.

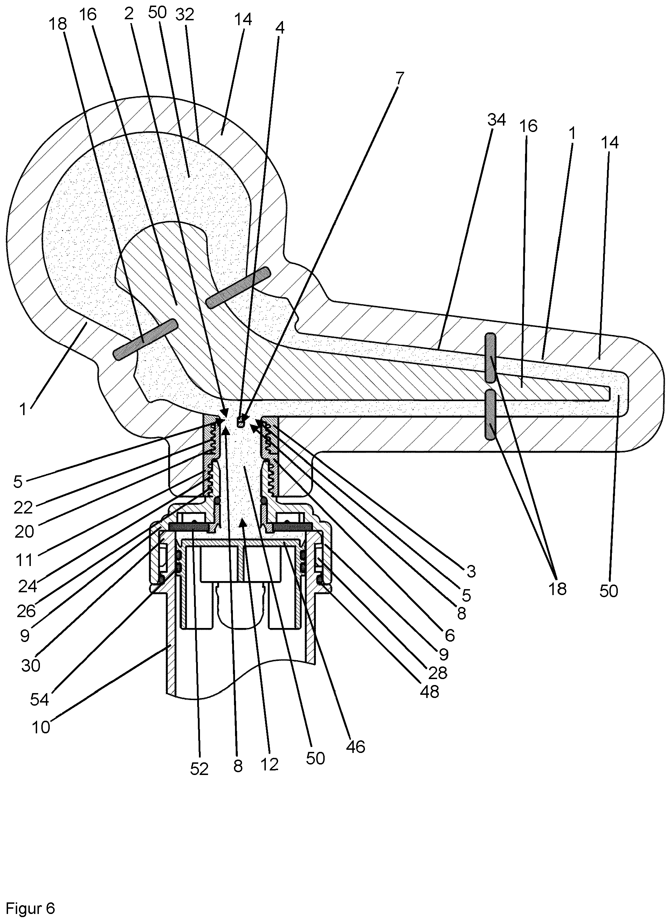

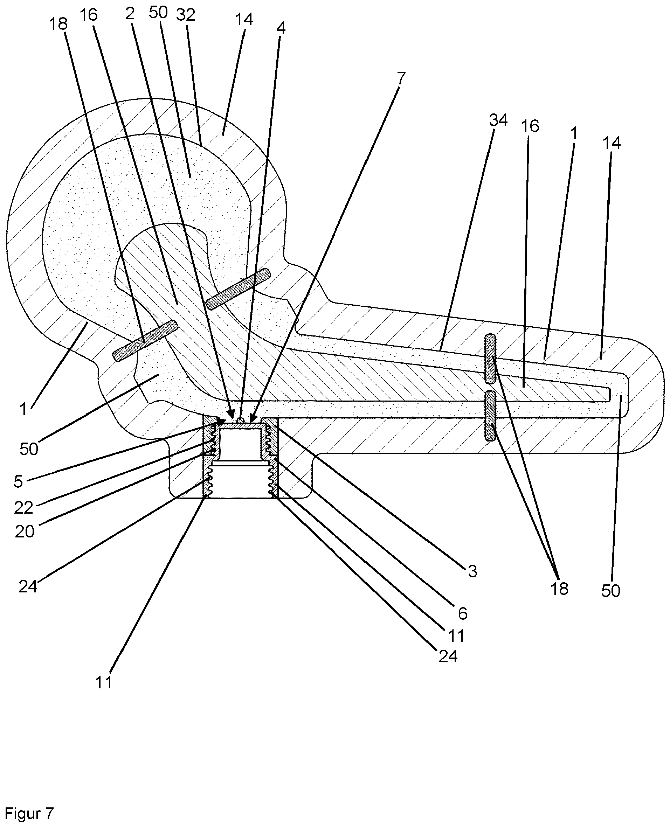

Irgendwann ist die Gießform 1 mit dem Knochenzementteig 50 gefüllt. Diese Situation ist in Figur 6 gezeigt. Luft oder Gas aus der Gießform 1 ist durch Entlüftungsöffnungen in der Gießform 1 entwichen. Durch Schließen des Ventils mit dem Hebel 38 wird der Knochenzementteig 50 abgeschert beziehungsweise abgeschnitten. Die Knochenzementkartusche 10 kann abgeschraubt und entfernt werden. Eventuell verbliebene dünne Verbindungen reißen oder brechen ohne weiteres ab. Diese Situation ist in Figur 7 dargestellt.

In diesem Zustand kann der Knochenzementteig 50 in der Gießform 1 ausgehärtet werden. Anschließend wird der so geformte Spacer aus der Gießform 1 entnommen. Die vorspringenden Haltestifte 18 können abgeschnitten werden. Ebenso kann ein durch den Ventilsitz 3 und die ersten Durchgänge 5 verursachter Anguss abgeschnitten und entfernt werden. Auch können Spitzen, die durch die Entlüftungsöffnungen verursacht werden, entfernt werden. Die Oberfläche des Spacers kann poliert werden und/oder beschichtet werden, beispielsweise mit Antibiotika.

Anstatt einer Gießform 1 zum Formen eines Hüftgelenkspacers kann ohne weiteres auch eine Gießform zum Formen eines anderen Spacers verwendet werden.

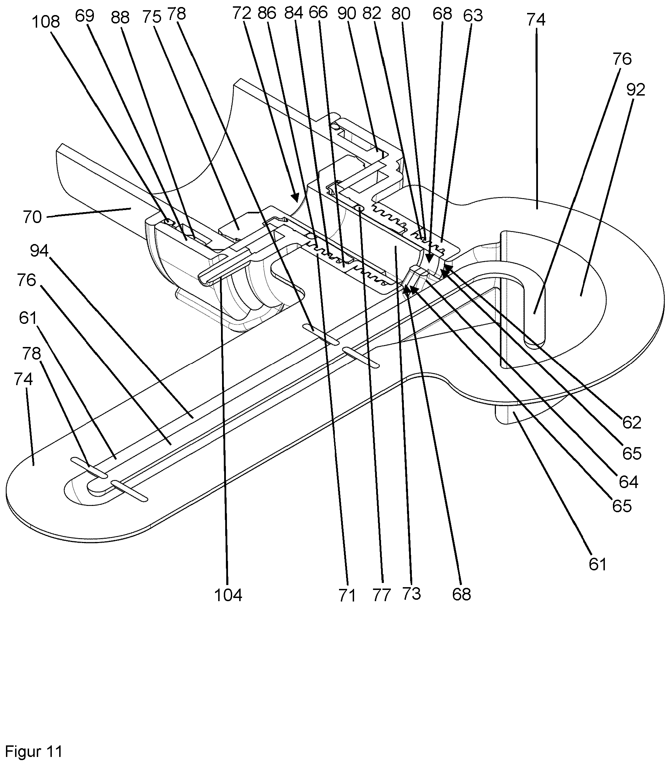

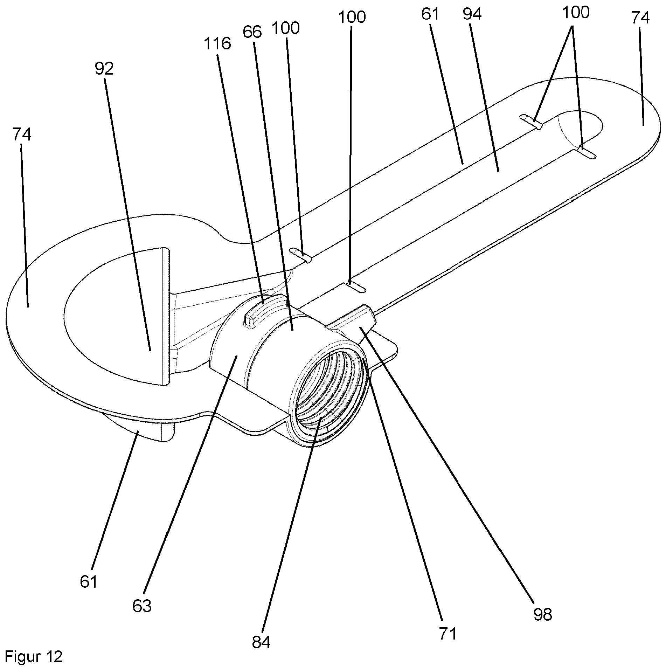

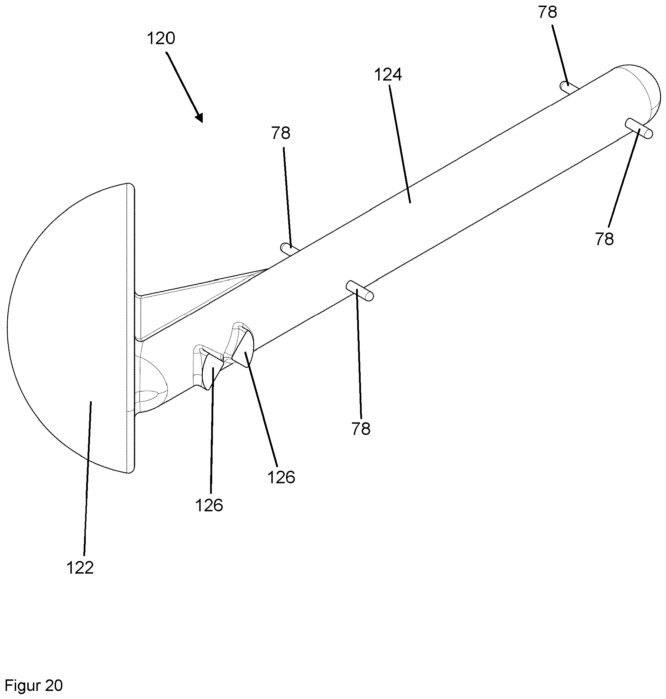

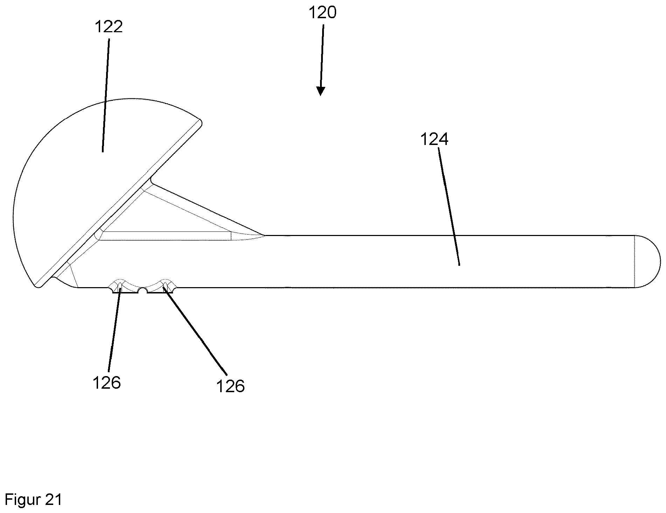

In den Figuren 11 bis 19 sind Abbildungen eines zweiten Ausführungsbeispiels einer erfindungsgemäßen Vorrichtung zur Herstellung eines Spacers für ein Schultergelenk und Teile davon in verschiedenen Ansichten gezeigt. Die Figuren 20 und 21 zeigen einen Schultergelenkspacer, der mit einer solchen zweiten erfindungsgemäßen Vorrichtung hergestellt wurde, als Resultat eines erfindungsgemäßen Verfahrens, dessen Verfahrensschritte chronologisch in den Figuren 12 bis 21 gezeigt sind.

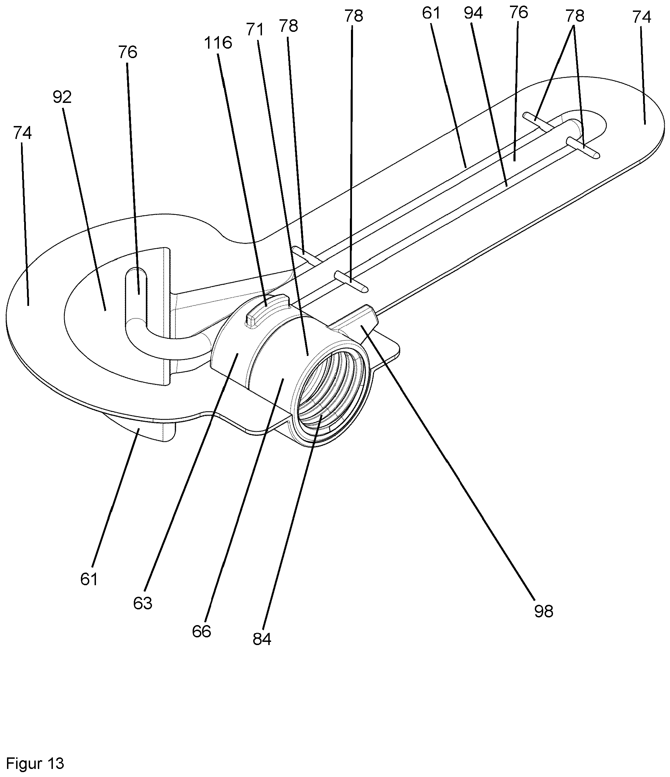

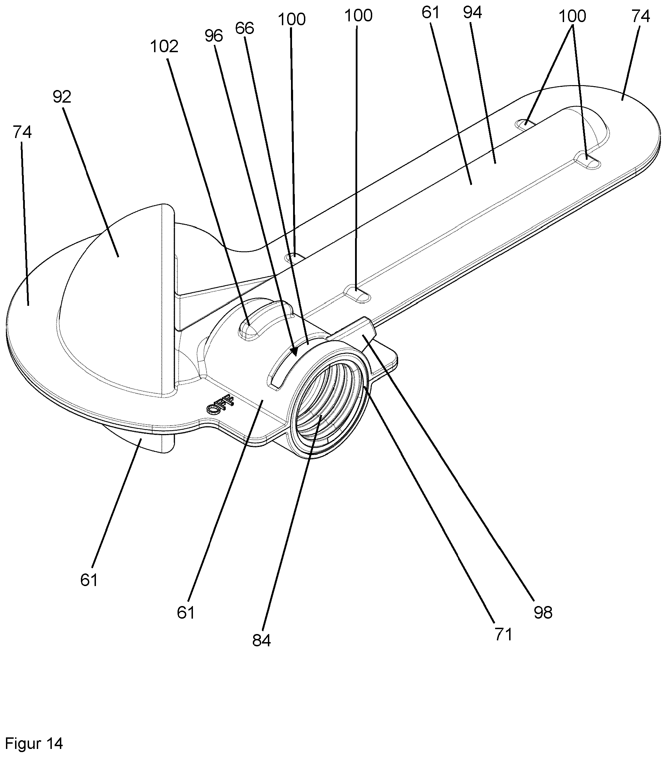

Die zweite erfindungsgemäße Vorrichtung ist zur Herstellung eines Spacers 120 für ein Schultergelenk geeignet und vorgesehen. Die Vorrichtung umfasst eine Gießform 61. Die Gießform 61 kann zweiteilig aufgebaut sein. In den Figuren 11 bis 13, 16, 17 und 19 ist jeweils nur einer der beiden Teile der Gießform 61 erkennbar. In den Figuren 14, 15 und 18 sind beiden Teile der Gießform 61 dargestellt. An einer Seite der Gießform 61 kann eine Einfüllöffnung 62 zum Einfüllen von Knochenzementteig 50 ausgeformt sein, die durch eine in beiden Teilen der Gießform 61 jeweils Halbkreisförmige zylindrische Öffnung definiert sein kann. In dieser Einfüllöffnung 62 kann ein Ventilsitz 63 angeordnet sein. Der Ventilsitz 63 kann mit einem Teil der Gießform 61 fest verbunden sein, so wie das in Figur 12 und 13 dargestellt ist. Zur besseren und dichteren Verbindung des Ventilsitzes 63 an die Gießform 61, kann der Ventilsitz 63 an seiner äußeren Oberfläche eine Strukturierung aufweisen, beispielsweise Längsrillen, die parallel zur Zylinderachse einer zylindrischen Außenwand des Ventilsitzes 63 angeordnet sind.

Der Ventilsitz 63 kann als ein Hohlzylinder ausgeführt sein, der auf einer in Richtung der Einfüllöffnung 62 ausgerichteten Kopfseite 64 bis auf zwei erste Durchführungen 65 geschlossen ist. Die beiden ersten Durchführungen 65 können viertelkreisförmig geformt sein und können vorzugsweise um 180° bezüglich der Zylinderachse des Ventilsitzes 63 gegeneinander verdreht angeordnet sein. Im Inneren des Ventilsitzes 63 kann ein gegen den Ventilsitz 63 axial verdrehbarer Ventilkörper 66 angeordnet sein. Der Ventilkörper 66 kann eine in Richtung der Kopfseite 64 des Ventilsitzes 63 ausgerichtete Dichtfläche 67 beziehungsweise Oberfläche aufweisen. Der Ventilkörper 66 kann als abgestufter Hohlzylinder aufgebaut sein, dessen vorderer Teil in den Ventilsitz 63 eingeschraubt oder eingesteckt sein kann.

In der Dichtfläche 67 können zwei zweite Durchführungen 68 angeordnet sein. Die beiden zweiten Durchführungen 68 können analog den ersten Durchführungen 65 viertelkreisförmig geformt sein und können vorzugsweise um 180° bezüglich der Zylinderachse des Ventilkörpers 66 gegeneinander verdreht beziehungsweise versetzt angeordnet sein. Der Ventilsitz 63 und der Ventilkörper 66 bilden zusammen ein Ventil der Vorrichtung. In den Ventilkörper 66 kann ein Adapterelement 69 zum flüssigkeitsdichten Verbinden einer Knochenzementkartusche 70 eingeschraubt sein. Die Knochenzementkartusche 70 und das Adapterelement 69 können Teil der erfindungsgemäßen Vorrichtung sein. Der Ventilkörper 66 kann an seiner offenen Seite, die von der Dichtfläche 67 abgewandt ist, als Anschluss 71 zum Verbinden des Adapterelements 69 ausgeformt sein.

Die Knochenzementkartusche 70 kann an ihrer Vorderseite eine Austragsöffnung 72 zum Austragen des Knochenzementteigs 50 aus der Knochenzementkartusche 70 aufweisen. Die Austragsöffnung 72 ist in dem Adapterelement 69 angeordnet. Im Inneren der Knochenzementkartusche 70 kann sich ein Austragsrohr 73 befinden, das im Innenraum der Knochenzementkartusche 70 mit einem Mischer 75 verbunden ist. Das Austragsrohr 73 ist in dem Adapterelement 69 axial beweglich geführt und drehbar gelagert. Durch Bewegen des Austragsrohrs 73 kann der Mischer 75 im Innenraum der Knochenzementkartusche 70 bewegt werden, um den Knochenzementteig 50 im Innenraum der Knochenzementkartusche 70 zu durchmischen. Vor dem Befestigen des Adapterelements 69 an dem Anschluss 71 kann das Austragsrohr 73 an einer Sollbruchstelle abgebrochen werden. Das Austragsrohr 73 kann über eine umlaufende Dichtung 77 gegen das Adapterelement 69 abgedichtet sein. Das Adapterelement 69 kann die Knochenzementkartusche 70 an ihrer Vorderseite bis auf die Austragsöffnung 72 und gegebenenfalls bis auf einen Vakuumanschluss 104 verschließen.

Die Gießform 61 kann kostengünstig aus Kunststofffolie gefertigt werden. Die Kunststofffolie kann mehrere Schichten aufweisen. Die beiden Teile der Gießform 61 können über Flansche 74 aneinander befestigt werden. Durch Verbinden der beiden Teile der Gießform 61 über die Flansche 74 kann die Gießform 61 nach außen verschlossen werden. In der Gießform 61 können Entlüftungsöffnungen (in den Figuren 11 bis 19 nicht zu sehen) angeordnet sein. Durch die Entlüftungsöffnungen kann Luft oder Gas aus dem Inneren der geschlossenen Gießform 61 entweichen, wenn ein Knochenzementteig 50 durch die Einfüllöffnung 62 in die Gießform 61 eingefüllt wird.

Im Inneren der Gießform 61 kann ein Metallkern 76 platziert werden. Der Metallkern 76 kann aus chirurgischem Stahl oder aus Titan bestehen. Der Metallkern 76 für den Schultergelenkspacer kann ein zu einem Haken gebogener zylindrischer Körper sein. Alternativ wäre es theoretisch auch möglich, den Metallkern 76 aus einem Kunststoff wie PMMA zu fertigen. Der Metallkern 76 kann über Haltestifte 78 mit der Gießform 61 verbunden werden. Der Metallkern 76 kann mit Hilfe der Haltestifte 78 von der Innenwand der Gießform 61 beabstandet sein, so dass der Knochenzementteig 50 den Metallkern 76 vollständig umfließen kann. Der Metallkern 76 bewirkt eine Stabilisierung des Spacers 120. Die Haltestifte 78 können aus PMMA bestehen. Dieses kann sich mit einem Knochenzementteig 50 aus PMMA irreversibel verbinden.

Der Ventilsitz 63 kann an seiner Innenseite ein Innengewinde 80 aufweisen. An der der Dichtfläche 67 zugewandten vorderen Hälfte des Ventilkörpers 66 kann der Ventilkörper 66 an seiner Außenseite ein zum Innengewinde 80 des Ventilsitzes 63 passendes Außengewinde 82 aufweisen. Der Ventilkörper 66 kann mit seinem Außengewinde 82 in das Innengewinde 80 des Ventilsitzes 63 eingeschraubt werden.

Durch Einschrauben des Ventilkörpers 66 in den Ventilsitz 63 bis zum Anschlag können die ersten Durchführungen 65 und die zweiten Durchführungen 68 in Überdeckung zueinander gebracht werden. Das Ventil befindet sich dann im geöffneten Zustand. Ein Knochenzementteig 50 kann in diesem geöffneten Zustand durch die ersten Durchführungen 65 und durch die zweiten Durchführungen 68 aus der Knochenzementkartusche 70 in die Gießform 61 fließen. Durch eine Vierteldrehung (um 90°) des Ventilkörpers 66 gegen den Ventilsitz 63, das heißt durch ein Herausschrauben des Ventilkörpers 66 aus dem Ventilsitz 63, können die ersten Durchführungen 65 und die zweiten Durchführungen 68 gegeneinander versetzt werden, so dass die Dichtfläche 67 des Ventilkörpers 66 die ersten Durchführungen 65 des Ventilsitzes 63 abdeckt und die geschlossenen Bereiche der Kopfseite 64 des Ventilsitzes 63 die zweiten Durchführungen 68 des Ventilkörpers 66 abdeckt. Das Ventil befindet sich dann im geschlossenen Zustand. Durch den geringen Hub des Ventilkörpers 66 gegen den Ventilsitz 63 bei einer Vierteldrehung, ist der zwischen dem Ventilkörper 66 und dem Ventilsitz 63 entstehende Spalt so schmal (weniger als 1 mm breit), dass ein normaler und erst recht ein hochviskoser Knochenzementteig 50 nicht durch den Spalt dringen können. Dies gilt insbesondere, weil der Knochenzementteig 50 im Spalt von seiner eigentlichen Flussrichtung um 90° abgelenkt wird.

In der Rückseite des Ventilkörpers 66 kann im Anschluss 71 ein Innengewinde 84 angeordnet sein. Das Adapterelement 69 weist an seiner Vorderseite ein zum Innengewinde 84 passendes Außengewinde 86 auf. Das Adapterelement 69 kann so in den Anschluss 71 des Ventilkörpers 66 eingeschraubt werden. Hierdurch kann eine flüssigkeitsdichte Verbindung der Knochenzementkartusche 70 zum Ventilkörper 66 und damit in die Gießform 61 erzeugt werden. Das Innengewinde 80 des Ventilsitzes 63, das Außengewinde 82 des Ventilkörpers 66, das Innengewinde 84 des Ventilkörpers 66 und das Außengewinde 86 des Adapterelements 69 können alle den gleichen Drehsinn aufweisen, das heißt, dass alle diese Gewinde Rechtsgewinde oder Linksgewinde sind. Dadurch kann das Ventil durch Einschrauben des Adapterelements 69 in den Anschluss 71 und gleichsinniges Weiterdrehen des Adapterelements 69 geöffnet werden. Gleichzeitig dichtet auch der Ventilkörper 66 gegen den Ventilsitz 63 ab.

Das Adapterelement 69 kann über ein Rastmittel 88 am Adapterelement 69 mit einer Gegenrastung 90 an einer zylindrischen Wandung der Knochenzementkartusche 70 verbunden werden oder sein. Zur Abdichtung kann eine umlaufende Dichtung 108 vorgesehen sein, die die zylindrische Wandung der Knochenzementkartusche 70 gegen das Adapterelement 69 abdichtet.

Die Gießform 61 kann einen Gelenkkopf-Formteil 92 zum Ausformen eines Kopfs 122 des Spacers 120 und einen Schaft-Formteil 94 zum Ausformen eines Schafts 124 des Spacers 120 aufweisen (siehe Figuren 20 und 21). Des Weiteren kann in der Gießform 61 im Bereich der Einfüllöffnung 62 eine Aussparung 96 für einen Hebel 98 des Ventilkörpers 66 angeordnet sein. Der Hebel 98 kann mit dem Ventilkörper 66 verbunden sein. Der Ventilkörper 66 kann mit dem Hebel 98 im Ventilsitz 63 gedreht werden. Die Aussparung 96 ist vorzugsweise genau so groß, dass den Ventilkörper 66 nur um maximal eine Vierteldrehung gegen den Ventilsitz 63 gedreht werden kann. Dadurch kann das Ventil mit Hilfe des Hebels 98 manuell von außen vom geöffneten Zustand in den geschlossenen Zustand beziehungsweise vom geschlossenen Zustand in den geöffneten Zustand überführt werden.

Im Bereich der Flansche 74 können in der Gießform Formen 100 für Kavitäten angeordnet sein, in denen die Haltestifte 78 angeordnet werden können. Ferner können in der Gießform 61 in den die Einfüllöffnung 62 begrenzenden Wandungen Formen 102 zur Ventilbefestigung angeordnet sein, die zur Aufnahme passender Vorsprünge 116 (siehe Figuren 12 und 13) an der zylindrischen Außenseite des Ventilsitzes 63 vorgesehen sind. Durch Eingreifen der Vorsprünge 116 in die Formen 102 zur Ventilbefestigung kann der Ventilsitz 63 nicht in der Einfüllöffnung 62 gedreht werden und es wird eine stabile Verbindung bereitgestellt.

In dem Adapterelement 69 kann ein Vakuumanschluss 104 angeordnet sein, mit dem ein Innenraum der Knochenzementkartusche 70, in dem der Knochenzementteig 50 gemischt wird, evakuiert werden kann. Dadurch kann der Knochenzementteig 50 unter Vakuum gemischt werden.

Im zylindrischen Innenraum der Knochenzementkartusche 70 kann ein Kolben 106 zum Austreiben des Knochenzementteigs 50 aus der Knochenzementkartusche 70 durch das Ventil in die Gießform 61 angeordnet sein. Hierzu kann der Kolben 106 an seiner Außenseite zylindrisch geformt und über zwei umlaufende Dichtungen 114 gegen den zylindrischen Innenraum abgedichtet sein. Durch Vortreiben des Kolbens 106 kann der Knochenzementteig 50 aus der Austragsöffnung 72 der Knochenzementkartusche 70 in beziehungsweise durch das geöffnete Ventil gepresst werden.

In dem Adapterelement 69 kann eine Porenscheibe 112 angeordnet sein. Die Porenscheibe 112 ist für den Knochenzementteig 50 und seine Ausgangskomponenten undurchlässig. Der Vakuumanschluss 104 kann von der Porenscheibe 112 abgedeckt sein. Hierdurch wird verhindert, dass ein Knochenzementpulver als Ausgangskomponente des Knochenzementteigs 50 in den Vakuumanschluss 104 eindringen kann.

Der Ablauf eines erfindungsgemäßen Verfahrens ist in den Figuren 12 bis 21 anhand der zweiten erfindungsgemäßen Vorrichtung dargestellt. Als erstes wird die geöffnete Gießform 61 bereitgestellt. Diese Situation ist in Figur 12 dargestellt. Dann kann der Metallkern 76 mit den Haltestiften 78 in der Gießform 61 positioniert werden. Hierzu können die Haltestifte 78 an einem Ende zwischen den beiden Teilen der Gießform 61 in den durch die Formen 100 gebildeten Kavitäten angeordnet und gehalten und mit dem anderen Ende in passende Bohrungen im Metallkern 76 angeordnet werden. Diese Situation ist in Figur 13 dargestellt.

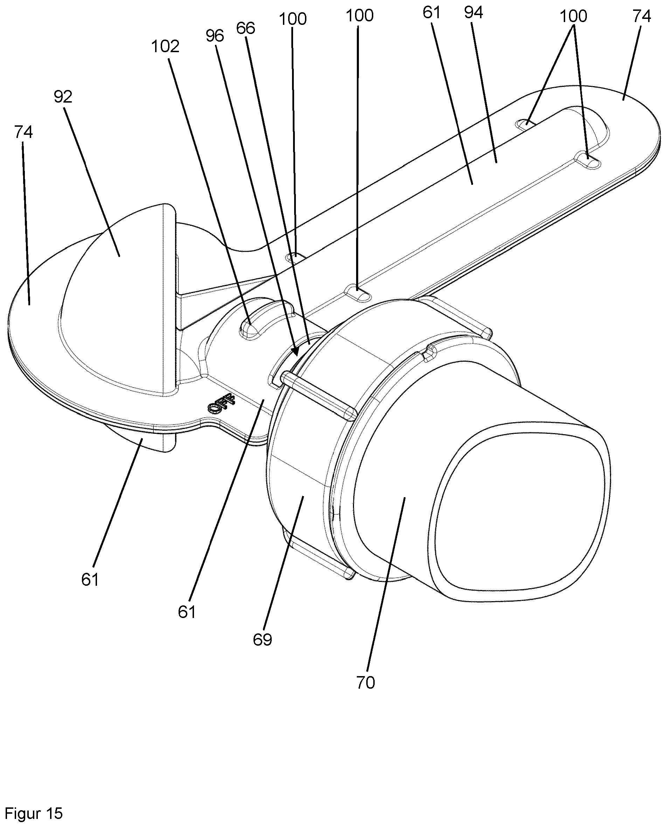

Die Gießform 61 kann anschließend geschlossen werden. Diese Situation ist in Figur 14 dargestellt. Ein Knochenzementteig 50 kann in der Knochenzementkartusche 70 unter Vakuum gemischt werden. Anschließend kann die Knochenzementkartusche 70 mit dem Adapterelement 69 in den Anschluss 71 des Ventilkörpers 66 geschraubt werden. Beim Einschrauben der Adapterelements 69 kann das Ventil in die geöffnete Stellung überführt werden, indem der Ventilkörper 66 bis zum Anschlag in den Ventilsitz 63 geschraubt wird. Diese Situation ist in Figur 15 dargestellt.

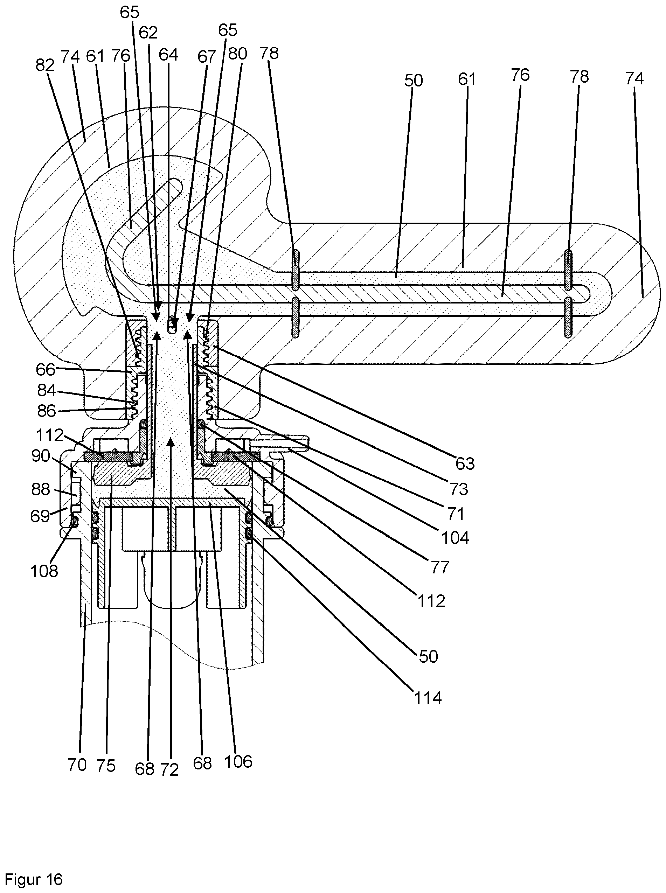

Anschließend wird durch Vortreiben des Kolbens 106 der Knochenzementteig 50 aus der Knochenzementkartusche 70 durch das Ventil und durch die sich in Deckung befindlichen ersten Durchführungen 65 und zweiten Durchführungen 68 in die Gießform 61 gepresst. Diese Situation ist in Figur 16 gezeigt. Durch Schließen des Ventils durch manuelles Bedienen des Hebels 98 und dadurch Drehen des Ventilkörpers 66 um eine Vierteldrehung gegen den Ventilsitz 63 kann zwischendurch eine neue Knochenzementkartusche 70 angesetzt werden, falls das Volumen des Knochenzementteigs 50 einer einzigen Knochenzementkartusche 70 nicht ausreicht, um die Gießform 61 vollständig zu füllen. Dabei kann der in der Gießform 61 enthaltene Knochenzementteig 50 nicht wieder ausfließen, das die ersten Durchgänge 65 und die zweiten Durchgänge 68 in der geschlossenen Stellung des Ventils abgedeckt sind und der Spalt dazwischen nicht ausreicht, um den viskosen Knochenzementteig 50 hindurchfließen lassen zu können.

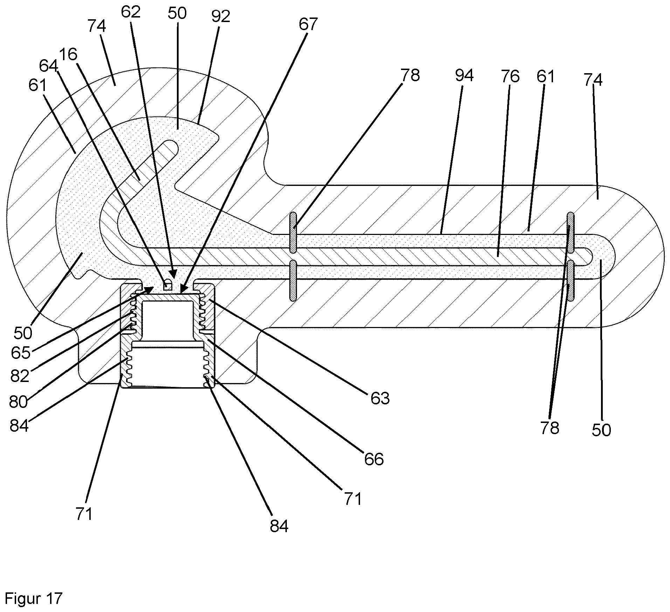

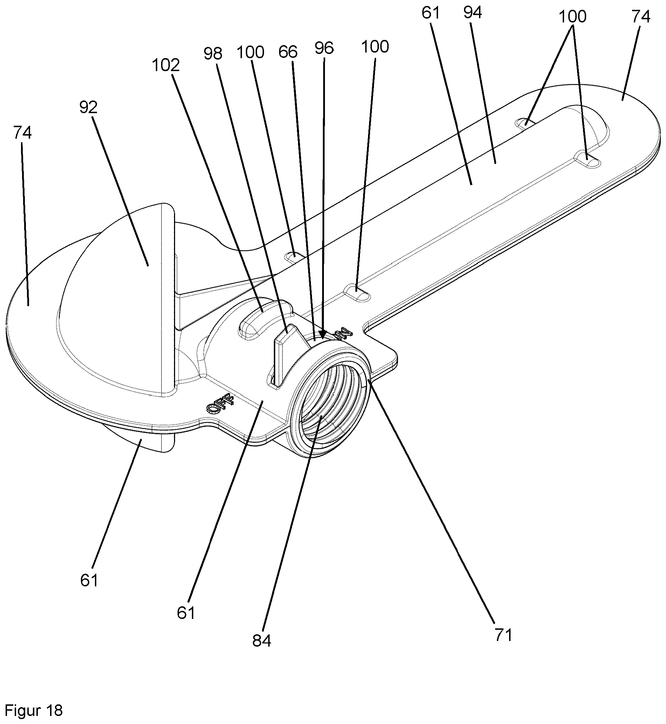

Irgendwann ist die Gießform 61 mit dem Knochenzementteig 50 gefüllt. Luft oder Gas aus der Gießform 1 ist durch Entlüftungsöffnungen in der Gießform 61 entwichen. Durch Schließen des Ventils mit dem Hebel 98 wird der Knochenzementteig 50 abgeschert beziehungsweise abgeschnitten. Die Knochenzementkartusche 70 kann abgeschraubt und entfernt werden. Eventuell verbliebene dünne Verbindungen reißen oder brechen ohne weiteres ab. Diese Situation ist in Figur 17 und Figur 18 dargestellt.

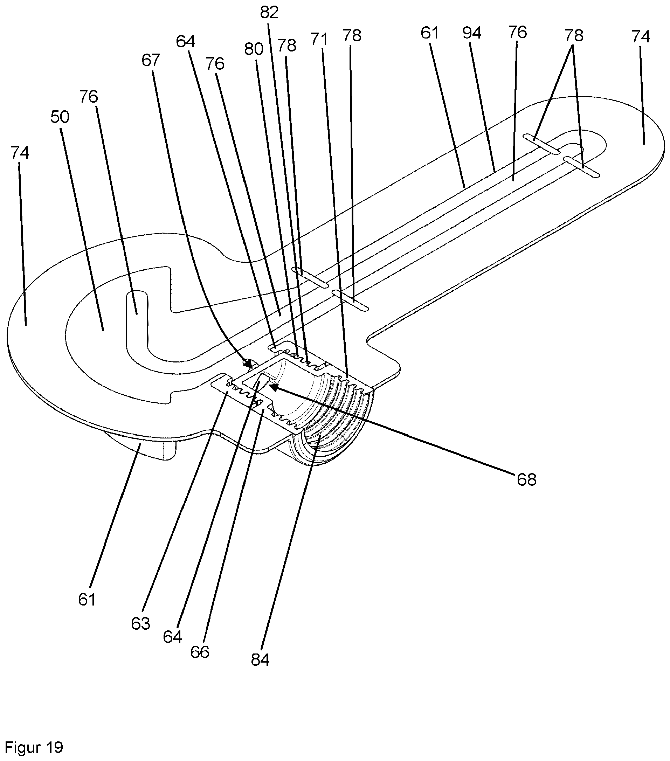

In diesem Zustand kann der Knochenzementteig 50 in der Gießform 61 ausgehärtet werden. Diese Situation ist in Figur 19 gezeigt. Anschließend wird der so geformte Spacer 120 aus der Gießform 1 entnommen. Der entnommene Spacer 120 ist in Figur 20 gezeigt. Die vorspringenden Haltestifte 78 können abgeschnitten werden. Diese Situation ist in Figur 21 gezeigt. Ebenso kann ein durch den Ventilsitz 63 und die ersten Durchgänge 65 verursachter Anguss 126 abgeschnitten und entfernt werden. Auch können Spitzen, die durch die Entlüftungsöffnungen verursacht werden, entfernt werden. Die Oberfläche des Spacers 120 kann poliert werden und/oder beschichtet werden, beispielsweise mit Antibiotika.

Anstatt einer Gießform 61 zum Formen eines Schultergelenkspacers kann ohne weiteres auch eine Gießform zum Formen eines anderen Spacers verwendet werden.

Die Figuren 22 bis 28 zeigen ein Ventil für eine erfindungsgemäße Vorrichtung zur Herstellung eines Spacers in geöffneter Stellung (Figuren 22 bis 24) und in geschlossener Stellung (Figuren 25 bis 28). Das Ventil entspricht den Ventilen der ersten erfindungsgemäßen Vorrichtung nach den Figuren 1 bis 10 und der zweiten erfindungsgemäßen Vorrichtung nach den Figuren 11 bis 19, ist aber auch mit anderen Gießformen zur Herstellung anderer Spacer einsetzbar.

Das Ventil hat einen Ventilsitz 163, der in einer Einfüllöffnung einer Gießform (nicht gezeigt) angeordnet werden kann. Der Ventilsitz 163 kann mit einem Teil der Gießform fest verbunden sein. Zur besseren und dichteren Verbindbarkeit des Ventilsitzes 163 mit einer Gießform kann der Ventilsitz 163 an seiner äußeren Oberfläche eine Strukturierung aufweisen, beispielsweise Längsrillen, die parallel zur Zylinderachse einer zylindrischen Außenwand des Ventilsitzes 163 angeordnet sind.

Der Ventilsitz 163 kann als ein Hohlzylinder ausgeführt sein, der auf einer Kopfseite 164 bis auf zwei erste Durchführungen 165 geschlossen ist. Die beiden ersten Durchführungen 165 können viertelkreisförmig geformt sein und können vorzugsweise um 180° bezüglich der Zylinderachse des Ventilsitzes 163 gegeneinander verdreht angeordnet sein. Im Inneren des Ventilsitzes 163 kann ein gegen den Ventilsitz 163 axial verdrehbarer Ventilkörper 166 angeordnet sein. Der Ventilkörper 166 kann eine in Richtung der Kopfseite 164 des Ventilsitzes 163 ausgerichtete Dichtfläche 167 beziehungsweise Oberfläche aufweisen. Der Ventilkörper 166 kann als abgestufter Hohlzylinder aufgebaut sein, dessen vorderer Teil in den Ventilsitz 163 eingeschraubt oder eingesteckt sein kann.

In der Dichtfläche 167 können zwei zweite Durchführungen 168 angeordnet sein. Die beiden zweiten Durchführungen 168 können analog den ersten Durchführungen 165 viertelkreisförmig geformt sein und können vorzugsweise um 180° bezüglich der Zylinderachse des Ventilkörpers 166 gegeneinander verdreht beziehungsweise versetzt angeordnet sein. Der Ventilsitz 163 und der Ventilkörper 166 bilden zusammen das Ventil einer erfindungsgemäßen Vorrichtung. In den Ventilkörper 166 kann ein Adapterelement (nicht gezeigt) zum flüssigkeitsdichten Verbinden einer Knochenzementkartusche (nicht gezeigt) eingeschraubt werden. Der Ventilkörper 166 kann an seiner offenen Seite, die von der Dichtfläche 167 abgewandt ist, als Anschluss 171 zum Verbinden eines Adapterelements ausgeformt sein.

Der Ventilsitz 163 kann an seiner Innenseite ein Innengewinde 180 aufweisen. An der der Dichtfläche 167 zugewandten vorderen Hälfte des Ventilkörpers 166 kann der Ventilkörper 166 an seiner Außenseite ein zum Innengewinde 180 des Ventilsitzes 163 passendes Außengewinde 182 aufweisen. Der Ventilkörper 166 kann mit seinem Außengewinde 182 in das Innengewinde 180 des Ventilsitzes 163 eingeschraubt werden.

Durch Einschrauben des Ventilkörpers 166 in den Ventilsitz 163 bis zum Anschlag können die ersten Durchführungen 165 und die zweiten Durchführungen 168 in Überdeckung zueinander gebracht werden. Das Ventil befindet sich dann im geöffneten Zustand. Ein Knochenzementteig kann in diesem geöffneten Zustand (siehe Figuren 22 bis 24) durch die ersten Durchführungen 165 und durch die zweiten Durchführungen 168 fließen. Durch eine Vierteldrehung (um 90°) des Ventilkörpers 166 gegen den Ventilsitz 163, das heißt durch ein Herausschrauben des Ventilkörpers 166 aus dem Ventilsitz 163, können die ersten Durchführungen 165 und die zweiten Durchführungen 168 gegeneinander versetzt werden, so dass die Dichtfläche 167 des Ventilkörpers 166 die ersten Durchführungen 165 des Ventilsitzes 163 abdeckt und die geschlossenen Bereiche der Kopfseite 164 des Ventilsitzes 163 die zweiten Durchführungen 168 des Ventilkörpers 166 abdeckt. Das Ventil befindet sich dann im geschlossenen Zustand (siehe Figuren 25 bis 28). Durch den geringen Hub des Ventilkörpers 166 gegen den Ventilsitz 163 bei einer Vierteldrehung, ist der zwischen dem Ventilkörper 166 und dem Ventilsitz 163 entstehende Spalt 220 so schmal (weniger als 1 mm breit), dass ein normaler und erst recht ein hochviskoser Knochenzementteig nicht durch den Spalt 220 dringen können (siehe Figur 28). Dies gilt insbesondere, weil der Knochenzementteig im Spalt 220 von seiner eigentlichen Flussrichtung um 90° abgelenkt wird.

In der Rückseite des Ventilkörpers 166 kann im Anschluss 171 ein Innengewinde 184 angeordnet sein. Ein Adapterelement (nicht gezeigt) kann so in den Anschluss 171 des Ventilkörpers 166 eingeschraubt werden. Das Innengewinde 180 des Ventilsitzes 163, das Außengewinde 182 des Ventilkörpers 166 und das Innengewinde 184 des Ventilkörpers 166 können alle den gleichen Drehsinn aufweisen, das heißt, dass alle diese Gewinde Rechtsgewinde oder Linksgewinde sind. Dadurch kann das Ventil durch Einschrauben eines Adapterelements in den Anschluss 171 und gleichsinniges Weiterdrehen des Adapterelements geöffnet werden. Gleichzeitig dichtet auch der Ventilkörper 166 gegen den Ventilsitz 163 ab.

Des Weiteren kann ein Hebel 198 am Ventilkörper 166 angeordnet sein. Der Ventilkörper 166 kann mit dem Hebel 198 im Ventilsitz 163 gedreht werden. Dadurch kann das Ventil mit Hilfe des Hebels 198 manuell von außen vom geöffneten Zustand in den geschlossenen Zustand beziehungsweise vom geschlossenen Zustand in den geöffneten Zustand überführt werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 61: Gießform
- 2, 62: Einfüllöffnung
- 3, 63, 163: Ventilsitz
- 4, 64, 164: Kopfseite
- 5, 65, 165: Durchführung
- 6, 66, 166: Ventilkörper
- 7, 67, 167: Dichtfläche
- 8, 68, 168: Durchführung
- 9, 69: Adapterelement
- 10, 70: Knochenzementkartusche
- 11, 71, 171: Anschluss
- 12, 72: Austragsöffnung
- 14, 74: Flansch
- 16, 76: Metallkern
- 18, 78: Haltestift
- 20, 80, 180: Innengewinde
- 22, 82, 182: Außengewinde
- 24, 84, 184: Innengewinde
- 26, 86: Außengewinde
- 28, 88: Rastmittel
- 30, 90: Gegenrastung
- 32, 92: Gelenkkopf-Formteil
- 34, 94: Schaft-Formteil
- 36, 96: Aussparung
- 38, 98, 198: Hebel
- 40, 100: Form für Kavitäten
- 42, 102: Form zur Ventilbefestigung
- 44, 104: Vakuumanschluss
- 46, 106: Kolben
- 48, 108: Dichtung
- 50: Knochenzementteig
- 52, 112: Porenscheibe
- 54, 114: Dichtung
- 56, 116, 216: Vorsprung
- 58: Bohrung
- 73: Austragsrohr
- 75: Mischer
- 77: Dichtung
- 120: Spacer
- 122: Kopf
- 124: Schaft
- 126: Anguss
- 220: Spalt

## Patentansprüche

1. Vorrichtung zum Herstellen eines Spacers (120) durch Aushärten von Knochenzementteig (50), wobei der Spacer (120) dazu vorgesehen ist, im medizinischen Bereich ein Gelenk oder einen Teil eines Gelenks umfassend eine artikulierende Oberfläche des Gelenks temporär zu ersetzen, insbesondere ein Hüftgelenk oder ein Schultergelenk temporär zu ersetzen, die Vorrichtung aufweisend eine Gießform (1, 61) zum Formen des Spacers (120) aus Knochenzementteig (50) mit wenigstens einer Einfüllöffnung (2, 62) zum Einfüllen eines Knochenzementteigs (50); einen Ventilsitz (3, 63, 163), der im Bereich der wenigstens einen Einfüllöffnung (2, 62) mit der Gießform (1, 61) verbunden ist, wobei der Ventilsitz (3, 63, 163) eine bereichsweise geschlossene Kopfseite (4, 64, 164) mit mindestens einer ersten Durchführung (5, 65, 165) aufweist, wobei die mindestens eine erste Durchführung (5, 65, 165) in die wenigstens eine Einfüllöffnung (2, 62) mündet;
einen Ventilkörper (6, 66, 166), der drehbar gegen den Ventilsitz (3, 63, 163) gelagert ist und der eine Dichtfläche (7, 67, 167) aufweist, wobei die Dichtfläche (7, 67, 167) in Richtung der bereichsweise geschlossenen Kopfseite (4, 64, 164) des Ventilsitzes (3, 63, 163) ausgerichtet ist, wobei in der Dichtfläche (7, 67, 167) mindestens eine zweite Durchführung (8, 68, 168) angeordnet ist;
wobei der Ventilsitz (3, 63, 163) und der Ventilkörper (6, 66, 166) zusammen ein Ventil bilden, wobei das Ventil durch Drehen des Ventilkörpers (6, 66, 166) gegen den Ventilsitz (3, 63, 163) reversibel in eine geöffnete Stellung und reversibel in eine geschlossene Stellung überführbar ist, wobei in der geöffneten Stellung des Ventils die mindestens eine erste Durchführung (5, 65, 165) des Ventilsitzes (3, 63, 163) und die mindestens eine zweite Durchführung (8, 68, 168) des Ventilkörpers (6, 66, 166) zumindest bereichsweise übereinanderliegen und eine für Knochenzementteig (50) durchlässige Verbindung durch das Ventil in die Gießform (1, 61) bereitstellen, wobei in der geschlossenen Stellung des Ventils die mindestens eine erste Durchführung (5, 65, 165) des Ventilsitzes (3, 63, 163) durch die Dichtfläche (7, 67, 167) des Ventilkörpers (6, 66, 166) abgedeckt ist, wobei die wenigstens eine Einfüllöffnung (2, 62) der Gießform (1, 61) in der geschlossenen Stellung des Ventils für Knochenzementteig (50) abgedeckt ist und wobei das Ventil auf der von der Gießform (1, 61) abgewandten Seite mit einem Anschluss (11, 71, 171) zum flüssigkeitsdichten Verbinden einer Knochenzementkartusche (10, 70) verbunden ist oder das Ventil einen solchen Anschluss (11, 71, 171) aufweist.

2. Vorrichtung nach Anspruch 1 **dadurch gekennzeichnet, dass**
der Ventilsitz (3, 63, 163) nicht verdrehbar gegen die Gießform (1, 61) mit der Gießform (1, 61) verbunden ist, bevorzugt der Ventilsitz (3, 63, 163) fest und/oder starr mit der Gießform (1, 61) verbunden ist, und/oder
das Ventil manuell bedienbar ist, bevorzugt manuell von außerhalb der Vorrichtung manuell bedienbar ist, wobei besonders bevorzugt der Ventilkörper (6, 66, 166) manuell gegen den Ventilsitz (3, 63, 163) drehbar ist und durch die Drehung das Ventil von der geschlossenen Stellung in die geöffnete Stellung und von der geöffneten Stellung in die geschlossene Stellung überführbar ist.

3. Vorrichtung nach einem der vorangehenden Ansprüche, **dadurch gekennzeichnet, dass**
die mindestens eine erste Durchführung (5, 65, 165) des Ventilsitzes (3, 63, 163) in der geschlossenen Stellung des Ventils mit der Dichtfläche (7, 67, 167) des Ventilkörpers (6, 66, 166) abgedeckt ist, wobei bevorzugt die bereichsweise geschlossene Kopfseite (4, 64, 164) des Ventilsitzes (3, 63, 163) und die Dichtfläche (7, 67, 167) des Ventilkörpers (6, 66, 166) maximal 2 mm voneinander beabstandet sind, besonders bevorzugt maximal 1 mm voneinander beabstandet sind, ganz besonders bevorzugt maximal 0,5 mm voneinander beabstandet sind.

4. Vorrichtung nach einem der vorangehenden Ansprüche, **dadurch gekennzeichnet, dass**
der Ventilkörper (6, 66, 166) um eine Drehachse gegen den Ventilsitz (3, 63, 163) drehbar gelagert ist, wobei die Drehachse senkrecht zur Dichtfläche (7, 67, 167) des Ventilkörpers (6, 66, 166) verläuft oder wobei die Drehachse entlang einer Rotationssymmetrieachse der Dichtfläche (7, 67, 167) des Ventilkörpers (6, 66, 166) verläuft, und/oder
der Ventilkörper (6, 66, 166) den Anschluss (11, 71, 171) zum flüssigkeitsdichten Verbinden einer Knochenzementkartusche (10, 70) aufweist oder mit einem solchen Anschluss (11, 71, 171) fest verbunden ist.

5. Vorrichtung nach einem der vorangehenden Ansprüche, **dadurch gekennzeichnet, dass**
die Vorrichtung ein Adapterelement (9, 69) aufweist, das mit einer Knochenzementkartusche (10, 70) verbunden ist oder verbindbar ist, wobei das Adapterelement (9, 69) lösbar und formschlüssig mit dem Anschluss (11, 71, 171) verbunden oder verbindbar ist, so dass ein Innenraum der Knochenzementkartusche (10, 70) über das Adapterelement (9, 69) für Knochenzementteig (50) durchlässig mit der mindestens einen zweiten Durchführung (8, 68, 168) im Ventilkörper (6, 66, 166) verbunden oder verbindbar ist, und
die Vorrichtung eine Knochenzementkartusche (10, 70) zum Mischen von Knochenzement-Ausgangskomponenten und zum Austragen von gemischtem Knochenzementteig (50) aus der Knochenzementkartusche (10, 70) heraus aufweist, bevorzugt eine Knochenzementkartusche (10, 70) zum Mischen von Polymethylmethacrylat-Knochenzement-Ausgangskomponenten und zum Austragen von gemischtem Polymethylmethacrylat-Knochenzementteig (50) aus der Knochenzementkartusche (10, 70) heraus aufweist, wobei besonders bevorzugt die Knochenzementkartusche (10, 70) die Knochenzement-Ausgangskomponenten zur Herstellung des Knochenzements in voneinander getrennten Bereichen beinhaltet.

6. Vorrichtung nach einem der vorangehenden Ansprüche, **dadurch gekennzeichnet, dass**
die Gießform (1, 61) aus einer Kunststofffolie besteht oder im Wesentlichen aus einer Kunststofffolie besteht oder
die Gießform (1, 61) aus zwei oder mehreren Kunststofffolien aufgebaut ist, die miteinander verschweißt oder verklebt sind, und wobei bevorzugt die Gießform (1, 61) aus PETG-Folie und/oder Polyamid-Folie und/oder PE-Folie gefertigt ist, und/oder die Summe aller Öffnungen der mindestens einen ersten Durchführung (5, 65, 165) in der geschlossenen Kopfseite (4, 64, 164) höchstens so groß ist wie die geschlossene Oberfläche der Kopfseite (4, 64, 164) und
dass die Summe aller Öffnungen der mindestens einen zweiten Durchführung (8, 68, 168) in der Dichtfläche (7, 67, 167) höchstens so groß ist wie die geschlossene Oberfläche der Dichtfläche (7, 67, 167).

7. Vorrichtung nach einem der vorangehenden Ansprüche, **dadurch gekennzeichnet, dass**
der Ventilsitz (3, 63, 163) ein Innengewinde (20, 80, 180) an der Innenseite aufweist und der Ventilkörper (6, 66, 166) ein passendes Außengewinde (22, 82, 182) an der Außenseite aufweist, so dass der Ventilkörper (6, 66, 166) in den Ventilsitz (3, 63, 163) schraubbar ist, und
der Anschluss (11, 71, 171) zum flüssigkeitsdichten Verbinden einer Knochenzementkartusche (10, 70) ein Innengewinde (24, 84, 184) im Ventilkörper (6, 66, 166) oder ein Außengewinde am Ventilkörper (6, 66, 166) umfasst, wobei vorzugsweise ein Adapterelement (9, 69) der Knochenzementkartusche (10, 70) oder an der Knochenzementkartusche (10, 70) ein zu dem Innengewinde (24, 84, 184) oder zu dem Außengewinde passendes Gegengewinde (26, 86) aufweist.

8. Vorrichtung nach Anspruch 7, **dadurch gekennzeichnet, dass** das Innengewinde (24, 84, 184) im Ventilkörper (6, 66, 166) oder das Außengewinde am Ventilkörper (6, 66, 166) ein Rechtsgewinde ist und das Ventil durch eine gleichsinnige Rechtsdrehung des Ventilkörpers (6, 66, 166) von der geschlossenen in die geöffnete Stellung überführbar ist und das Ventil durch eine gegensinnige Linksdrehung des Ventilkörpers (6, 66, 166) von der geöffneten in die geschlossene Stellung überführbar ist oder
das Innengewinde (24, 84, 184) im Ventilkörper (6, 66, 166) oder das Außengewinde am Ventilkörper (6, 66, 166) ein Linksgewinde ist und das Ventil durch eine gleichsinnige Linksdrehung des Ventilkörpers (6, 66, 166) von der geschlossenen in die geöffnete Stellung überführbar ist und das Ventil durch eine gegensinnige Rechtsdrehung des Ventilkörpers (6, 66, 166) von der geöffneten in die geschlossene Stellung überführbar ist oder
das Innengewinde (20, 80, 180) des Ventilsitzes (3, 63, 163) und das Innengewinde (24, 84, 184) und das Außengewinde (22, 82, 182) des Ventilkörpers (6, 66, 166) alle Linksgewinde oder alle Rechtsgewinde sind, wobei vorzugsweise auch ein Außengewinde (26, 86) eines Adapterelements (9, 69) zum flüssigkeitsdichten Verbinden einer Knochenzementkartusche (10, 70) an den Anschluss (11, 71, 171) den gleichen Drehsinn hat.

9. Vorrichtung nach einem der vorangehenden Ansprüche, **dadurch gekennzeichnet, dass**
die mindestens eine erste Durchführung (5, 65, 165) in der bereichsweise geschlossenen Kopfseite (4, 64, 164) die gleiche Größe und Gestalt hat wie die mindestens eine zweite Durchführung (8, 68, 168) in der Dichtfläche (7, 67, 167) und/oder
die mindestens eine erste Durchführung (5, 65, 165) in der bereichsweise geschlossenen Kopfseite (4, 64, 164) zwei erste Durchführungen (5, 65, 165) sind und die mindestens eine zweite Durchführung (8, 68, 168) in der Dichtfläche (7, 67, 167) zwei zweite Durchführungen (8, 68, 168) sind, wobei bevorzugt die zwei ersten Durchführungen (5, 65, 165) in gegenüberliegend angeordneten Kreissegmentvierteln bezüglich der Drehachse des Ventilkörpers (6, 66, 166) im Ventilsitz (3, 63, 163) angeordnet sind und die zwei zweiten Durchführungen (8, 68, 168) in gegenüberliegend angeordneten Kreissegmentvierteln bezüglich der Drehachse des Ventilkörpers (6, 66, 166) in der Dichtfläche (7, 67, 167) angeordnet sind, und/oder an der Dichtfläche (7, 67, 167) des Ventilkörpers (6, 66, 166) ein Bund angeordnet ist, der sich auf einem Rand des Ventilsitzes (3, 63, 163) abstützt oder an der bereichsweise geschlossenen Kopfseite (4, 64, 164) des Ventilsitzes (3, 63, 163) ein Bund angeordnet ist, der sich auf einem Rand des Ventilkörpers (6, 66, 166) abstützt.

10. Vorrichtung nach einem der vorangehenden Ansprüche, **dadurch gekennzeichnet, dass**
an dem Ventilkörper (6, 66, 166) ein Hebel (38, 98, 198) angeordnet ist, der eine radiale Erstreckung bezüglich der Drehachse des Ventilkörpers (6, 66, 166) aufweist,
wobei vorzugsweise der Hebel (38, 98, 198) durch eine Aussparung (36, 96) in der Gießform (1, 61) oder in dem Ventilsitz (3, 63, 163) ragt, wobei die Aussparung (36, 96) in der Gießform (1, 61) gegebenenfalls im Bereich der Verbindung zum Ventilsitz (3, 63, 163) angeordnet ist, wobei die Aussparung (36, 96) derart dimensioniert ist, dass sich das Ventil durch Drehen des Ventilkörpers (6, 66, 166) im Ventilsitz (3, 63, 163) mittels des Hebels (38, 98, 198) von der geöffneten Stellung in die geschlossene Stellung und umgekehrt überführen lässt, wobei besonders bevorzugt die Aussparung (36, 96) derart dimensioniert ist, dass sich der Ventilkörper (6, 66, 166) maximal um 90° gegen den Ventilsitz (3, 63, 163) drehen lässt, und/oder
der Ventilkörper (6, 66, 166) und der Ventilsitz (3, 63, 163) aus Kunststoff gefertigt sind, insbesondere aus thermoplastischem Kunststoff gefertigt sind, wobei bevorzugt der Ventilsitz (3, 63, 163) mit einer Wandung der Gießform (1, 61) verklebt oder verschweißt ist.

11. Vorrichtung nach einem der vorangehenden Ansprüche, **dadurch gekennzeichnet, dass**
die Gießform (1, 61) ausgehend von einem Innenraum der Gießform (1, 61) mindestens drei oder vier Kavitäten zur Aufnahme von Haltestiften (18, 78) aufweist, wobei vorzugsweise die Gießform (1, 61) zweiteilig ist und die Kavitäten in Rändern oder in Flanschen (14, 74) zumindest eines Teils der zweiteiligen Gießform (1, 61) angeordnet sind, und
die Vorrichtung einen Metallkern (16, 76) aufweist, der in der Gießform (1, 61) anzuordnen ist, wobei bevorzugt der Metallkern (16, 76) Bohrungen (58) zur Aufnahme von Haltestiften (18, 78) besitzt, wobei besonders bevorzugt die Bohrungen (58) nicht in einem Bereich der Gießform (1, 61) zum Formen einer Gleitfläche des Spacers (120) angeordnet sind, ganz besonders bevorzugt die Bohrungen (58) in einem Bereich der Gießform (1, 61) zum Formen eines Schafts (124) des Spacers (120) angeordnet sind, und/oder
in der Gießform (1, 61) zumindest eine Entlüftungsöffnung vorgesehen ist, durch die Luft oder Gas aus dem Inneren der Gießform (1, 61) entweichen kann, wobei bevorzugt wenigstens eine der zumindest eine Entlüftungsöffnung, besonders bevorzugt jede der zumindest einen Entlüftungsöffnung, in einem Bereich der Gießform (1, 61) angeordnet ist, die eine Gleitfläche oder einen Gelenkkopf (122) des Spacers (120) formt.

12. Verfahren zur Herstellung eines Spacers (120) zum temporären Ersetzen eines Gelenks oder eines Teils eines Gelenks, insbesondere eines Hüftgelenks oder eines Schultergelenks, umfassend eine artikulierende Oberfläche des Gelenks, wobei das Verfahren mit einer Vorrichtung nach einem der Ansprüche 1 bis 11 durchgeführt wird,
das Verfahren aufweisend die folgenden chronologischen Schritte:
A) Flüssigkeitsdichtes Verbinden einer Knochenzementkartusche (10, 70) mit dem Anschluss (11, 71, 171) der Vorrichtung;
B) Einpressen von Knochenzementteig (50) aus der Knochenzementkartusche (10, 70) durch das Ventil in der geöffneten Stellung hindurch in die Gießform (1, 61);
C) Drehen des Ventilkörpers (6, 66, 166) gegen den Ventilsitz (3, 63, 163) und dadurch Überführen des Ventils in die geschlossene Stellung und Abscheren des Knochenzementteigs (50) an der mindestens einen ersten Durchführung (5, 65, 165) in der bereichsweise geschlossenen Kopfseite (4, 64, 164) des Ventilsitzes (3, 63, 163) durch die Drehung des Ventilkörpers (6, 66, 166) gegen den Ventilsitz (3, 63, 163);
D) Lösen der Knochenzementkartusche (10, 70) von dem Anschluss (11, 71, 171);
E) Aushärten des Knochenzementteigs (50) in der Gießform (1, 61); und
F) Entnehmen des so geformten und ausgehärteten Spacers (120) aus der Gießform (1,61).

13. Verfahren nach Anspruch 12, **dadurch gekennzeichnet, dass**
nach Schritt D) und vor Schritt E) die folgenden Zwischenschritte erfolgen:
D2) Flüssigkeitsdichtes Verbinden einer neuen Knochenzementkartusche (10, 70) mit dem Anschluss (11, 71, 171) der Vorrichtung, wobei in der neuen Knochenzementkartusche (10, 70) Knochenzementteig (50) oder Ausgangskomponenten zur Herstellung des Knochenzementteigs (50) enthalten ist oder sind;
D3) Drehen des Ventilkörpers (6, 66, 166) gegen den Ventilsitz (3, 63, 163) und dadurch Überführen des Ventils in die geöffnete Stellung;
D4) Einpressen des Knochenzementteigs (50) aus der neuen Knochenzementkartusche (10, 70) durch das Ventil in der geöffneten Stellung hindurch in die Gießform (1, 61);
D5) Drehen des Ventilkörpers (6, 66, 166) gegen den Ventilsitz (3, 63, 163) und dadurch Überführen des Ventils in die geschlossene Stellung und Abscheren des Knochenzementteigs (50) an der mindestens einen ersten Durchführung (5, 65, 165) in der bereichsweise geschlossenen Kopfseite (4, 64, 164) des Ventilsitzes (3, 63, 163) durch die Drehung des Ventilkörpers (6, 66, 166) gegen den Ventilsitz (3, 63, 163); und
D6) Lösen der neuen Knochenzementkartusche (10, 70) von dem Anschluss (11, 71, 171);
wobei vorzugsweise die Schritte D2) bis D6) einmal oder mehrfach mit jeweils neuen Knochenzementkartuschen (10, 70), die Knochenzementteig (50) oder dessen Ausgangskomponenten enthalten, wiederholt werden, bis die Gießform (1, 61) vollständig oder nach Bedarf mit Knochenzementteig (50) gefüllt ist.

14. Verfahren nach Anspruch 12 oder 13, **dadurch gekennzeichnet, dass** vor Schritt B), und vorzugsweise vor Schritt A), der Knochenzementteig (50) in der Knochenzementkartusche (10, 70) aus einer Monomerflüssigkeit und aus einem Zementpulver gemischt wird, wobei bevorzugt gegebenenfalls vor Schritt D3), vorzugsweise vor Schritt D2), der Knochenzementteig (50) in der neuen Knochenzementkartusche (10, 70) aus einer Monomerflüssigkeit und aus einem Zementpulver gemischt wird, und/oder
zum flüssigkeitsdichten Verbinden der Knochenzementkartusche (10, 70) und/oder der neuen Knochenzementkartusche (10, 70) mit dem Anschluss (11, 71, 171) die Knochenzementkartusche (10, 70) oder die neue Knochenzementkartusche (10, 70) in den Anschluss (11, 71, 171) hineingedreht oder eingeschraubt wird und zum Lösen der Knochenzementkartusche (10, 70) und/oder der neuen Knochenzementkartusche (10, 70) von dem Anschluss (11, 71, 171) die Knochenzementkartusche (10, 70) oder die neue Knochenzementkartusche (10, 70) aus dem Anschluss (11, 71, 171) herausgedreht oder herausgeschraubt wird, und/oder
das Drehen des Ventilkörpers (6, 66, 166) gegen den Ventilsitz (3, 63, 163) durch ein Schrauben des Ventilkörpers (6, 66, 166) in dem Ventilsitz (3, 63, 163) erfolgt oder durch ein manuelles Drehen des Ventilkörpers (6, 66, 166) gegen Ventilsitz (3, 63, 163) erfolgt, wobei bevorzugt das manuelle Drehen durch Bedienen eines sich radial von Ventilkörper (6, 66, 166) weg erstreckenden Hebels (38, 98, 198) erfolgt, der sich durch eine Aussparung (36, 96) in der Gießform (1, 61) oder in dem Ventilsitz (3, 63, 163) erstreckt.

15. Verfahren nach einem der Ansprüche 12 bis 14, **dadurch gekennzeichnet, dass** das Einpressen des Knochenzementteigs (50) aus der Knochenzementkartusche (10, 70) oder der neuen Knochenzementkartusche (10, 70) durch Eindrücken eines Kolbens (46, 106) in einen Innenraum der Knochenzementkartusche (10, 70) erfolgt, und/oder
vor Schritt B), und vorzugsweise vor Schritt A), ein Metallkern (16, 76) innerhalb der Gießform (1, 61) angeordnet wird, wobei bevorzugt der Metallkern (16, 76) über mehrere Haltestifte (18, 78) von einer Innenwand der Gießform (1, 61) beabstandet wird, wobei besonders bevorzugt die mehreren Haltestifte (18, 78) in Bohrungen (58) im Metallkern (16, 76) und in Kavitäten zur Aufnahme von Haltestiften (18, 78) in der Innenwand der Gießform (1, 61) befestigt werden.

## Claims

1. A device for producing a spacer (120) by curing bone cement paste (50), wherein the spacer (120) is provided in the medical field for temporarily replacing a joint or part of a joint comprising an articulating surface of the joint, in particular for temporarily replacing a hip joint or a shoulder joint, the device having
a casting mold (1, 61) for molding the spacer (120) from bone cement paste (50), the casting mold (1, 61) having at least one filling opening (2, 62) for introducing a bone cement paste (50);
a valve seat (3, 63, 163), the valve seat (3, 63, 163) being connected to the casting mold (1, 61) in the region of the at least one filling opening (2, 62), wherein the valve seat (3, 63, 163) has a regionally closed head side (4, 64, 164) with at least one first feed-through (5, 65, 165), wherein the at least one first feed-through (5, 65, 165) opens into the at least one filling opening (2, 62);
a valve body (6, 66, 166) which is mounted so as to be rotatable relative to the valve seat (3, 63, 163) and which has a sealing face (7, 67, 167), wherein the sealing face (7, 67, 167) is oriented in the direction of the regionally closed head side (4, 64, 164) of the valve seat (3, 63, 163), wherein at least one second feed-through (8, 68, 168) is arranged in the sealing face (7, 67, 167);
wherein the valve seat (3, 63, 163) and the valve body (6, 66, 166) together form a valve, wherein the valve is reversibly transferable into an open position and into a closed position by rotation of the valve body (6, 66, 166) relative to the valve seat (3, 63, 163), wherein, in the open position of the valve, the at least one first feed-through (5, 65, 165) of the valve seat (3, 63, 163) and the at least one second feed-through (8, 68, 168) of the valve body (6, 66, 166) are located above one another at least in places and provide a connection permeable to bone cement (50) through the valve into the casting mold (1, 61), wherein, in the closed position of the valve, the at least one first feed-through (5, 65, 165) of the valve seat (3, 63, 163) is covered by the sealing face (7, 67, 167) of the valve body (6, 66, 166), wherein, in the closed position of the valve, the at least one filling opening (2, 62) of the casting mold (1, 61) is covered and closed for bone cement paste (50) and wherein the valve is connected on the side remote from the casting mold (1, 61) to a port (11, 71, 171) for liquid-tight connection of a bone cement cartridge (10, 70) or the valve has such a port (11, 71, 171).

2. The device according to Claim 1, **characterized in that**
the valve seat (3, 63, 163) is connected to the casting mold (1, 61) so as not to be rotatable relative to the casting mold (1, 61), preferably the valve seat (3, 63, 163) is firmly and/or rigidly connected to the casting mold (1, 61), and/or
the valve is manually operable, preferably manually operable from outside the device, wherein the valve body (6, 66, 166) is particularly preferably manually rotatable relative to the valve seat (3, 63, 163) and the valve is transferable by rotation from the closed position into the open position and from the open position into the closed position.

3. The device according to any one of the preceding claims, **characterized in that**, in the closed position of the valve, the at least one first feed-through (5, 65, 165) of the valve seat (3, 63, 163) is covered by the sealing face (7, 67, 167) of the valve body (6, 66, 166), wherein the regionally closed head side (4, 64, 164) of the valve seat (3, 63, 163) and the sealing face (7, 67, 167) of the valve body (6, 66, 166) are preferably spaced apart from one another by a maximum of 2 mm, particularly preferably by a maximum of 1 mm and very particularly preferably by a maximum of 0.5 mm.

4. The device according to any one of the preceding claims, **characterized in that** the valve body (6, 66, 166) is mounted so as to be rotatable about an axis of rotation relative to the valve seat (3, 63, 163), wherein the axis of rotation extends perpendicular to the sealing face (7, 67, 167) of the valve body (6, 66, 166) or wherein the axis of rotation extends along an axis of rotational symmetry of the sealing face (7, 67, 167) of the valve body (6, 66, 166), and/or
the valve body (6, 66, 166) has the port (11, 71, 171) for liquid-tight connection of a bone cement cartridge (10, 70) or is firmly connected to such a port (11, 71, 171).

5. The device according to any one of the preceding claims, **characterized in that** the device has an adapter element (9, 69) which is connected or connectable to a bone cement cartridge (10, 70), wherein the adapter element (9, 69) is detachably and interlockingly connected or connectable to the port (11, 71, 171), such that an interior of the bone cement cartridge (10, 70) is connected or connectable permeably for bone cement paste (50) via the adapter element (9, 69) to the at least one second feed-through (8, 68, 168) in the valve body (6, 66, 166), and
the device has a bone cement cartridge (10, 70) for mixing bone cement starting components and for delivering mixed bone cement paste (50) from the bone cement cartridge (10, 70) and preferably has a bone cement cartridge (10, 70) for mixing polymethyl methacrylate bone cement starting components and for delivering mixed polymethyl methacrylate bone cement paste (50) from the bone cement cartridge (10, 70), wherein the bone cement cartridge (10, 70) particularly preferably contains the bone cement starting components for producing the bone cement in mutually separate regions.

6. The device according to any one of the preceding claims, **characterized in that** the casting mold (1, 61) consists of a plastics film or substantially of a plastics film or the casting mold (1, 61) is constructed from two or more plastics films, which are welded or adhesively bonded together, and wherein the casting mold (1, 61) is preferably fabricated from PETG film and/or polyamide film and/or PE film, and/or
the sum of all the openings of the at least one first feed-through (5, 65, 165) in the closed head side (4, 64, 164) is at most as large as the closed surface of the head side (4, 64, 164) and
the sum of all the openings of the at least one second feed-through (8, 68, 168) in the sealing face (7, 67, 167) is at most as large as the closed surface of the sealing face (7, 67, 167).

7. The device according to any one of the preceding claims, **characterized in that** the valve seat (3, 63, 163) has an inner thread (20, 80, 180) on the inside and the valve body (6, 66, 166) has a matching outer thread (22, 82, 182) on the outside, such that the valve body (6, 66, 166) is able to be screwed into the valve seat (3, 63, 163), and the port (11, 71, 171) comprises, for liquid-tight connection of a bone cement cartridge (10, 70), an inner thread (24, 84, 184) in the valve body (6, 66, 166) or an outer thread on the valve body (6, 66, 166), wherein preferably an adapter element (9, 69) of the bone cement cartridge (10, 70) or on the bone cement cartridge (10, 70) has a mating thread (26, 86) matching the inner thread (24, 84, 184) or the outer thread.

8. The device according to Claim 7, **characterized in that** the inner thread (24, 84, 184) in the valve body (6, 66, 166) or the outer thread on the valve body (6, 66, 166) is a right-hand thread and the valve is transferable from the closed to the open position by equidirectional rightward rotation of the valve body (6, 66, 166) and the valve is transferable from the open to the closed position by contradirectional leftward rotation of the valve body (6, 66, 166) or
the inner thread (24, 84, 184) in the valve body (6, 66, 166) or the outer thread on the valve body (6, 66, 166) is a left-hand thread and the valve is transferable from the closed to the open position by equidirectional leftward rotation of the valve body (6, 66, 166) and the valve is transferable from the open to the closed position by contradirectional rightward rotation of the valve body (6, 66, 166) or
the inner thread (20, 80, 180) of the valve seat (3, 63, 163) and the inner thread (24, 84, 184) and the outer thread (22, 82, 182) of the valve body (6, 66, 166) are all left-hand threads or all right-hand threads, wherein an outer thread (26, 86) of an adapter element (9, 69) preferably also has the same direction of rotation for liquid-tight connection of a bone cement cartridge (10, 70) to the port (11, 71, 171).

9. The device according to any one of the preceding claims, **characterized in that** the at least one first feed-through (5, 65, 165) in the regionally closed head side (4, 64, 164) has the same size and shape as the at least one second feed-through (8, 68, 168) in the sealing face (7, 67, 167) and/or
the at least one first feed-through (5, 65, 165) in the regionally closed head side (4, 64, 164) is two first feed-throughs (5, 65, 165) and the at least one second feed-through (8, 68, 168) in the sealing face (7, 67, 167) is two second feed-throughs (8, 68, 168), wherein the two first feed-throughs (5, 65, 165) are preferably arranged in the valve seat (3, 63, 163) in quadrants arranged opposingly with regard to the axis of rotation of the valve body (6, 66, 166) and the two second feed-throughs (8, 68, 168) are arranged in the sealing face (7, 67, 167) in quadrants arranged opposingly with regard to the axis of rotation of the valve body (6, 66, 166), and/or
a collar is arranged on the sealing face (7, 67, 167) of the valve body (6, 66, 166), which collar rests on an edge of the valve seat (3, 63, 163) or a collar is arranged on the regionally closed head side (4, 64, 164) of the valve seat (3, 63, 163), which collar rests on an edge of the valve body (6, 66, 166).

10. The device according to any one of the preceding claims, **characterized in that** a lever (38, 98, 198) is arranged on the valve body (6, 66, 166), which lever has a radial extent with regard to the axis of rotation of the valve body (6, 66, 166), wherein preferably the lever (38, 98, 198) projects through an orifice (36, 96) in the casting mold (1, 61) or in the valve seat (3, 63, 163), wherein the orifice (36, 96) in the casting mold (1, 61) is optionally arranged in the region of the connection to the valve seat (3, 63, 163), wherein the orifice (36, 96) is dimensioned such that the valve may be transferred from the open position into the closed position and vice versa by rotation of the valve body (6, 66, 166) in the valve seat (3, 63, 163) by means of the lever (38, 98, 198), wherein the orifice (36, 96) is particularly preferably dimensioned such that the valve body (6, 66, 166) may be rotated by a maximum of 90° relative to the valve seat (3, 63, 163), and/or
the valve body (6, 66, 166) and the valve seat (3, 63, 163) are fabricated of plastics, in particular of thermoplastics, wherein the valve seat (3, 63, 163) is preferably adhesively bonded or welded to a wall of the casting mold (1, 61).

11. The device according to any one of the preceding claims, **characterized in that** the casting mold (1, 61) has at least three or four cavities, starting from an inner chamber of the casting mold (1, 61), for receiving retaining pins (18, 78), wherein the casting mold (1, 61) is preferably in two parts and the cavities are preferably arranged in edges or flanges (14, 74) of at least one part of the two-part casting mold (1, 61), and
the device has a metal core (16, 76) for arrangement in the casting mold (1, 61), wherein the metal core (16, 76) preferably has bores (58) for receiving retaining pins (18, 78), wherein the bores (58) are particularly preferably not arranged in a region of the casting mold (1, 61) for molding a sliding surface of the spacer (120), the bores (58) very particularly preferably being arranged in a region of the casting mold (1, 61) for molding a stem (124) of the spacer (120), and/or
at least one vent opening is provided in the casting mold (1, 61), through which air or gas can escape from the interior of the casting mold (1, 61), wherein preferably at least one of the at least one vent opening, particularly preferably each of the at least one vent opening, is arranged in a region of the casting mold (1, 61) which forms a sliding surface or a joint head (122) of the spacer (120).

12. A method for producing a spacer (120) for temporarily replacing a joint or part of a joint, in particular a hip joint or a shoulder joint, comprising an articulating surface of the joint, wherein the method is carried out with the device according to any one of Claims 1 to 11, the method having the following chronological steps:
A) connecting a bone cement cartridge (10, 70) to the port (11, 71, 171) of the device in liquid-tight manner;
B) injecting bone cement paste (50) from the bone cement cartridge (10, 70) through the valve in the open position into the casting mold (1, 61);
C) rotating the valve body (6, 66, 166) relative to the valve seat (3, 63, 163) and so transferring the valve into the closed position and shearing off the bone cement paste (50) at the at least one first feed-through (5, 65, 165) in the regionally closed head side (4, 64, 164) of the valve seat (3, 63, 163) by rotating the valve body (6, 66, 166) relative to the valve seat (3, 63, 163);
D) detaching the bone cement cartridge (10, 70) from the port (11, 71, 171);
E) curing the bone cement paste (50) in the casting mold (1, 61); and
F) removing the resultant molded and cured spacer (120) from the casting mold (1, 61).

13. The method according to Claim 12, **characterized in that** the following intermediate steps proceed after step D) and before step E):
D2) connecting a new bone cement cartridge (10, 70) to the port (11, 71, 171) of the device in liquid-tight manner, wherein bone cement paste (50) or starting components for producing the bone cement paste (50) is/are present in the new bone cement cartridge (10, 70);
D3) rotating the valve body (6, 66, 166) relative to the valve seat (3, 63, 163) and so transferring the valve into the open position;
D4) injecting the bone cement paste (50) from the new bone cement cartridge (10, 70) through the valve in the open position into the casting mold (1, 61);
D5) rotating the valve body (6, 66, 166) relative to the valve seat (3, 63, 163) and so transferring the valve into the closed position and shearing off the bone cement paste (50) at the at least one first feed-through (5, 65, 165) in the regionally closed head side (4, 64, 164) of the valve seat (3, 63, 163) by rotating the valve body (6, 66, 166) relative to the valve seat (3, 63, 163); and
D6) detaching the new bone cement cartridge (10, 70) from the port (11, 71, 171); wherein steps D2) to D6) are preferably repeated once or multiple times with in each case new bone cement cartridges (10, 70) which contain bone cement paste (50) or the starting components thereof until the casting mold (1, 61) is filled completely or as required with bone cement paste (50).

14. The method according to Claim 12 or 13, **characterized in that** the bone cement paste (50) is mixed before step B), and preferably before step A), in the bone cement cartridge (10, 70) from a monomer liquid and a cement powder, wherein, optionally before step D3) and preferably before step D2), the bone cement paste (50) is preferably mixed in the new bone cement cartridge (10, 70) from a monomer liquid and a cement powder, and/or
the bone cement cartridge (10, 70) and/or the new bone cement cartridge (10, 70) is rotated or screwed into the port (11, 71, 171) for liquid-tight connection of the bone cement cartridge (10, 70) and/or the new bone cement cartridge (10, 70) to the port (11, 71, 171) and, for detaching the bone cement cartridge (10, 70) and/or the new bone cement cartridge (10, 70) from the port (11, 71, 171), the bone cement cartridge (10, 70) or the new bone cement cartridge (10, 70) is rotated out of or unscrewed from the port (11, 71, 171), and/or
the valve body (6, 66, 166) is rotated relative to the valve seat (3, 63, 163) by screwing the valve body (6, 66, 166) in the valve seat (3, 63, 163) or by manually rotating the valve body (6, 66, 166) relative to the valve seat (3, 63, 163), wherein manual rotation preferably proceeds by operation of a lever (38, 98, 198) extending radially away from the valve body (6, 66, 166) and extending through an orifice (36, 96) in the casting mold (1, 61) or in the valve seat (3, 63, 163).

15. The method according to any one of Claims 12 to 14, **characterized in that** injection of the bone cement paste (50) from the bone cement cartridge (10, 70) or the new bone cement cartridge (10, 70) proceeds by pushing a piston (46, 106) into an interior of the bone cement cartridge (10, 70), and/or
a metal core (16, 76) is arranged within the casting mold (1, 61) before step B) and preferably before step A), wherein the metal core (16, 76) is preferably spaced from an internal wall of the casting mold (1, 61) via a plurality of retaining pins (18, 78), wherein the plurality of retaining pins (18, 78) are particularly preferably fastened in bores (58) in the metal core (16, 76) and in cavities for receiving retaining pins (18, 78) in the internal wall of the casting mold (1, 61).

## Revendications

1. Dispositif pour la fabrication d'un espaceur (120) par le durcissement de pâte de ciment osseux (50), l'espaceur (120) étant conçu pour remplacer temporairement, dans le domaine médical, une articulation ou une partie d'une articulation comprenant une surface articulée de l'articulation, en particulier pour remplacer temporairement une articulation de la hanche ou une articulation de l'épaule, le dispositif présentant un moule de coulée (1, 61) pour la formation de l'espaceur (120) à partir de pâte de ciment osseux (50) comprenant au moins une ouverture de remplissage (2, 62) pour le remplissage d'une pâte de ciment osseux (50) ; un siège de soupape (3, 63, 163), lequel est connecté au moule de coulée (1, 61) dans la zone de l'au moins une ouverture de remplissage (2, 62), le siège de soupape (3, 63, 163) présentant un côté tête (4, 64, 164) fermé par zones comprenant au moins un premier passage (5, 65, 165), l'au moins un premier passage (5, 65, 165) débouchant dans l'au moins une ouverture de remplissage (2, 62) ;
un corps de soupape (6, 66, 166), lequel est monté tournant par rapport au siège de soupape (3, 63, 163) et lequel présente une surface d'étanchéité (7, 67, 167), la surface d'étanchéité (7, 67, 167) étant orientée dans la direction du côté de tête (4, 64, 164) fermé par zones du siège de soupape (3, 63, 163), au moins un deuxième passage (8, 68, 168) étant disposé dans la surface d'étanchéité (7, 67, 167) ;
le siège de soupape (3, 63, 163) et le corps de soupape (6, 66, 166) formant ensemble une soupape, la soupape pouvant être transférée de manière réversible dans une position ouverte et de manière réversible dans une position fermée par rotation du corps de soupape (6, 66, 166) par rapport au siège de soupape (3, 63, 163), dans la position ouverte de la soupape l'au moins un premier passage (5, 65, 165) du siège de soupape (3, 63, 163) et l'au moins un deuxième passage (8, 68, 168) du corps de soupape (6, 66, 166) étant au moins par zones l'un au-dessus de l'autre et mettant à disposition une connexion perméable pour de la pâte de ciment osseux (50) à travers la soupape dans le moule de coulée (1, 61), dans la position fermée de la soupape l'au moins un premier passage (5, 65, 165) du siège de soupape (3, 63, 163) étant recouvert par la surface d'étanchéité (7, 67, 167) du corps de soupape (6, 66, 166), l'au moins une ouverture de remplissage (2, 62) du moule de coulée (1, 61) étant couverte pour de la pâte de ciment osseux (50) dans la position fermée de la soupape, et la soupape étant connectée sur le côté opposé au moule de coulée (1, 61) à un raccord (11, 71, 171) pour la connexion imperméable aux liquides d'une cartouche de ciment osseux (10, 70) ou la soupape présentant un tel raccord (11, 71, 171).

2. Dispositif selon la revendication 1, **caractérisé en ce que**
le siège de soupape (3, 63, 163) est connecté au moule de coulée (1, 61) de manière non tournante par rapport au moule de coulée (1, 61), de préférence le siège de soupape (3, 63, 163) est connecté de manière fixe et/ou rigide au moule de coulée (1, 61), et/ou la soupape peut être actionnée manuellement, de préférence peut être actionnée manuellement depuis l'extérieur du dispositif, le corps de soupape (6, 66, 166) pouvant de manière particulièrement préférée tourner manuellement par rapport au siège de soupape (3, 63, 163) et la soupape pouvant être transférée, par la rotation, de la position fermée à la position ouverte et de la position ouverte à la position fermée.

3. Dispositif selon l'une quelconque des revendications précédentes, **caractérisé en ce que** l'au moins un premier passage (5, 65, 165) du siège de soupape (3, 63, 163) est recouvert par la surface d'étanchéité (7, 67, 167) du corps de soupape (6, 66, 166) dans la position fermée de la soupape, le côté de tête (4, 64, 164) fermé par zones du siège de soupape (3, 63, 163) et la surface d'étanchéité (7, 67, 167) du corps de soupape (6, 66, 166) étant de préférence espacés au maximum de 2 mm l'un de l'autre, étant encore plus préférablement espacés au maximum de 1 mm l'un de l'autre, étant de manière particulièrement préférée espacés au maximum de 0,5 mm l'un de l'autre.

4. Dispositif selon l'une quelconque des revendications précédentes, **caractérisé en ce que** le corps de soupape (6, 66, 166) est monté de manière rotative autour d'un axe de rotation par rapport au siège de soupape (3, 63, 163), l'axe de rotation s'étendant perpendiculaire à la surface d'étanchéité (7, 67, 167) du corps de soupape (6, 66, 166) ou l'axe de rotation s'étendant le long d'un axe de symétrie en rotation de la surface d'étanchéité (7, 67, 167) du corps de soupape (6, 66, 166), et/ou
le corps de soupape (6, 66, 166) présente le raccord (11, 71, 171) pour la connexion étanche aux liquides d'une cartouche de ciment osseux (10, 70) ou est connecté de manière fixe à un tel raccord (11, 71, 171).

5. Dispositif selon l'une quelconque des revendications précédentes, **caractérisé en ce que** le dispositif présente un élément adaptateur (9, 69), lequel est ou peut être connecté à une cartouche de ciment osseux (10, 70), l'élément adaptateur (9, 69) étant ou pouvant être connecté de manière amovible et par liaison de forme au raccord (11, 71, 171), de sorte qu'un espace intérieur de la cartouche de ciment osseux (10) soit ou puisse être connecté par le biais de l'élément adaptateur (9, 69) de manière perméable pour la pâte de ciment osseux (50) à l'au moins un deuxième passage (8, 68, 168) dans le corps de soupape (6, 66, 166), et
le dispositif présente une cartouche de ciment osseux (10, 70) pour le mélange de composants initiaux de ciment osseux et pour la distribution de pâte de ciment osseux (50) mélangée hors de la cartouche de ciment osseux (10, 70), présente de préférence une cartouche de ciment osseux (10, 70) pour le mélange de composants initiaux de ciment osseux de poly(méthacrylate de méthyle) et pour la distribution de pâte de ciment osseux de poly(méthacrylate de méthyle) (50) mélangée hors de la cartouche de ciment osseux (10), la cartouche de ciment osseux (10, 70) contenant de manière particulièrement préférée les composants initiaux de ciment osseux pour la fabrication du ciment osseux dans des zones séparées les unes des autres.

6. Dispositif selon l'une quelconque des revendications précédentes, **caractérisé en ce que** le moule de coulée (1, 61) est composé d'une feuille de plastique ou est composé essentiellement d'une feuille de plastique ou
le moule de coulée (1, 61) est constitué de deux ou plusieurs feuilles de plastique, lesquelles sont soudées ou collées les unes aux autres, et le moule de coulée (1, 61) étant fabriqué de préférence en feuille de PETG et/ou en feuille de polyamide et/ou en feuille de PE, et/ou la somme de toutes les ouvertures de l'au moins un premier passage (5, 65, 165) dans le côté de tête (4, 64, 164) fermé est au plus aussi grande que la surface fermée du côté de tête (4, 64, 164) et
**en ce que** la somme de toutes les ouvertures de l'au moins un deuxième passage (8, 68, 168) dans la surface d'étanchéité (7, 67, 167) est au plus aussi grande que la surface fermée de la surface d'étanchéité (7, 67, 167).

7. Dispositif selon l'une quelconque des revendications précédentes, **caractérisé en ce que** le siège de soupape (3, 63, 163) présente un filetage interne (20, 80, 180) sur la face interne et le corps de soupape (6, 66, 166) présente un filetage externe (22, 82, 182) correspondant sur la face externe, de sorte que le corps de soupape (6, 66, 166) puisse être vissé dans le siège de soupape (3, 63, 163), et
le raccord (11, 71, 171) pour la connexion étanche aux liquides d'une cartouche de ciment osseux (10, 70) comprend un filetage interne (24, 84, 184) dans le corps de soupape (6, 66, 166) ou un filetage externe sur le corps de soupape (6, 66, 166), un élément adaptateur (9, 69) de la cartouche de ciment osseux (10, 70) ou sur la cartouche de ciment osseux (10, 70) présentant de préférence un contre-filetage (26, 86) correspondant au filetage interne (24, 84, 184) ou au filetage externe.

8. Dispositif selon la revendication 7, **caractérisé en ce que**
le filetage interne (24, 84, 184) dans le corps de soupape (6, 66, 166) ou le filetage externe sur le corps de soupape (6, 66, 166) est un filetage à droite et la soupape peut être transférée par une rotation de même sens vers la droite du corps de soupape (6, 66, 166) de la position fermée à la position ouverte et la soupape peut être transférée par une rotation en sens opposé vers la gauche du corps de soupape (6, 66, 166) de la position ouverte à la position fermée ou
le filetage interne (24, 84, 184) dans le corps de soupape (6, 66, 166) ou le filetage externe sur le corps de soupape (6, 66, 166) est un filetage à gauche et la soupape peut être transférée par une rotation de même sens vers la gauche du corps de soupape (6, 66, 166) de la position fermée à la position ouverte et la soupape peut être transférée par une rotation en sens opposé vers la droite du corps de soupape (6, 66, 166) de la position ouverte à la position fermée ou
le filetage interne (20, 80, 180) du siège de soupape (3, 63, 163) et le filetage interne (24, 84, 184) et le filetage externe (22, 82, 182) du corps de soupape (6, 66, 166) sont tous des filetages à gauche ou tous des filetages à droite, un filetage externe (26, 86) d'un élément adaptateur (9, 69) pour la connexion étanche aux liquides d'une cartouche de ciment osseux (10, 70) au raccord (11, 71, 171) ayant de préférence le même sens de rotation.

9. Dispositif selon l'une quelconque des revendications précédentes, **caractérisé en ce que** l'au moins un premier passage (5, 65, 165) dans le côté de tête (4, 64, 164) fermé par zones a la même taille et forme que l'au moins un deuxième passage (8, 68, 168) dans la surface d'étanchéité (7, 67, 167) et/ou
l'au moins un premier passage (5, 65, 165) dans le côté de tête (4, 64, 164) fermé par zones sont deux premiers passages (5, 65, 165) et l'au moins un deuxième passage (8, 68, 168) dans la surface d'étanchéité (7, 67, 167) sont deux deuxièmes passages (8, 68, 168), les deux premiers passages (5, 65, 165) étant de préférence disposés dans le siège de soupape (3, 63, 163) dans des quarts de segment de cercle disposés à l'opposé l'un de l'autre par rapport à l'axe de rotation du corps de soupape (6, 66, 166) et les deux deuxièmes passages (8, 68, 168) étant disposés dans la surface d'étanchéité (7, 67, 167) dans des quarts de segment de cercle disposés à l'opposé l'un de l'autre par rapport à l'axe de rotation du corps de soupape (6, 66, 166), et/ou
un épaulement est disposé sur la surface d'étanchéité (7, 67, 167) du corps de soupape (6, 66, 166), lequel s'appuie sur un bord du siège de soupape (3, 63, 163,) ou un épaulement est disposé sur le côté de tête (4, 64, 164) fermé par zones du siège de soupape (3, 63, 163), lequel s'appuie sur un bord du corps de soupape (6, 66, 166).

10. Dispositif selon l'une quelconque des revendications précédentes, **caractérisé en ce que** un levier (38, 98, 198) est disposé sur le corps de soupape (6, 66, 166), lequel présente une extension radiale par rapport à l'axe de rotation du corps de soupape (6, 66, 166), le levier (38, 98, 198) faisant saillie de préférence à travers un évidement (36, 96) dans le moule de coulée (1, 61) ou dans le siège de soupape (3, 63, 163), l'évidement (36, 96) dans le moule de coulée (1, 61) étant disposé éventuellement dans la zone de la connexion au siège de soupape (3, 63, 163), l'évidement (36, 96) étant dimensionné de façon que la soupape puisse être transférée par rotation du corps de soupape (6, 66, 166) dans le siège de soupape (3, 63, 163) au moyen du levier (38, 98, 198) de la position ouverte à la position fermée et vice versa, l'évidement (36, 96) étant encore plus préférablement dimensionné de façon que le corps de soupape (6, 66, 166) puisse être tourné au maximum de 90° par rapport au siège de soupape (3, 63, 163) et/ou
le corps de soupape (6, 66, 166) et le siège de soupape (3, 63, 163) étant fabriqués en plastique, en particulier fabriqués en plastique thermoplastique, le siège de soupape (3, 63, 163) étant de préférence collé ou soudé à une paroi du moule de coulée (1, 61).

11. Dispositif selon l'une quelconque des revendications précédentes, **caractérisé en ce que** le moule de coulée (1, 61) présente, à partir d'un espace intérieur du moule de coulée (1, 61), au moins trois ou quatre cavités pour la réception de chevilles de maintien (18, 78), le moule de coulée (1, 61) étant de préférence en deux parties et les cavités étant disposées dans des bords ou dans des brides (14, 74) d'au moins une partie du moule de coulée (1, 61) en deux parties, et
le dispositif présente un noyau métallique (16, 76), lequel doit être disposé dans le moule de coulée (1, 61), le noyau métallique (16, 76) possédant de préférence des alésages (58) pour la réception de chevilles de maintien (18, 78), les alésages (58) n'étant pas encore plus préférablement disposés dans une zone du moule de coulée (1, 61) destinée à la formation d'une surface de glissement de l'espaceur (120), les alésages (58) étant de manière particulièrement préférée disposés dans une zone du moule de coulée (1, 61) destinée à la formation d'une tige (124) de l'espaceur (120), et/ou
au moins une ouverture de ventilation est prévue dans le moule de coulée (1, 61), à travers laquelle de l'air ou du gaz peut échapper de l'intérieur du moule de coulée (1, 61), de préférence au moins une de l'au moins une ouverture de ventilation, encore plus préférablement chacune de l'au moins une ouverture de ventilation, étant disposée dans une zone du moule de coulée (1, 61) qui forme une surface de glissement ou une tête d'articulation (122) de l'espaceur (120).

12. Procédé pour la fabrication d'un espaceur (120) pour le remplacement temporaire d'une articulation ou d'une partie d'une articulation, en particulier d'une articulation de la hanche ou d'une articulation de l'épaule, comprenant une surface articulée de l'articulation, le procédé étant effectué avec un dispositif selon l'une quelconque des revendications 1 à 11, le procédé présentant les étapes chronologiques suivantes :
A) connexion étanche aux liquides d'une cartouche de ciment osseux (10, 70) au raccord (11, 71, 171) du dispositif ;
B) injection de pâte de ciment osseux (50) de la cartouche de ciment osseux (10, 70) à travers la soupape dans la position ouverte dans le moule de coulée (1, 61) ;
C) rotation du corps de soupape (6, 66, 166) par rapport au siège de soupape (3, 63, 163) et ainsi transfert de la soupape dans la position fermée et cisaillement de la pâte de ciment osseux (50) au niveau de l'au moins un premier passage (5, 65, 165) dans le côté de tête (4, 64, 164) fermé par zones du siège de soupape (3, 63, 163) par la rotation du corps de soupape (6, 66, 166) par rapport au siège de soupape (3, 63, 163) ;
D) détachement de la cartouche de ciment osseux (10, 70) du raccord (11, 71, 171) ;
E) durcissement de la pâte de ciment osseux (50) dans le moule de coulée (1, 61) ; et
F) extraction de l'espaceur (120) ainsi formé et durci du moule de coulée (1, 61).

13. Procédé selon la revendication 12, **caractérisé en ce que**
les étapes intermédiaires suivantes sont effectuées après l'étape D) et avant l'étape E) :
D2) connexion étanche aux liquides d'une nouvelle cartouche de ciment osseux (10, 70) au raccord (11, 71, 171) du dispositif, de la pâte de ciment osseux (50) ou des composants initiaux pour la fabrication de la pâte de ciment osseux (50) étant contenue ou contenus dans la nouvelle cartouche de ciment osseux (10, 70) ;
D3) rotation du corps de soupape (6, 66, 166) par rapport au siège de soupape (3, 63, 163) et ainsi transfert de la soupape dans la position ouverte ;
D4) injection de la pâte de ciment osseux (50) de la nouvelle cartouche de ciment osseux (10, 70) à travers la soupape dans la position ouverte dans le moule de coulée (1, 61) ;
D5) rotation du corps de soupape (6, 66, 166) par rapport au siège de soupape (3, 63, 163) et ainsi transfert de la soupape dans la position fermée et cisaillement de la pâte de ciment osseux (50) au niveau de l'au moins un premier passage (5, 65, 165) dans le côté de tête (4, 64, 164) fermé par zones du siège de soupape (3, 63, 163) par la rotation du corps de soupape (6, 66, 166) par rapport au siège de soupape (3, 63, 163) ; et
D6) détachement de la nouvelle cartouche de ciment osseux (10, 70) du raccord (11, 71, 171);
les étapes D2) à D6) étant de préférence répétées une fois ou plusieurs fois, avec respectivement de nouvelles cartouches de ciment osseux (10, 70), lesquelles contiennent de la pâte de ciment osseux (50) ou ses composants initiaux, jusqu'à ce que le moule de coulée (1, 61) soit rempli complètement ou selon les besoins de pâte de ciment osseux (50).

14. Procédé selon la revendication 12 ou 13, **caractérisé en ce que** avant l'étape B), et de préférence avant l'étape A), la pâte de ciment osseux (50) dans la cartouche de ciment osseux (10, 70) est mélangée à partir d'un liquide de monomère et d'une poudre de ciment, de préférence le cas échéant, avant l'étape D3), de préférence avant l'étape D2), la pâte de ciment osseux (50) dans la nouvelle cartouche de ciment osseux (10, 70) étant mélangée à partir d'un liquide de monomère et d'une poudre de ciment, et/ou pour la connexion étanche aux liquides de la cartouche de ciment osseux (10, 70) et/ou de la nouvelle cartouche de ciment osseux (10, 70) au raccord (11, 71, 171), la cartouche de ciment osseux (10, 70) ou la nouvelle cartouche de ciment osseux (10, 70) est tournée ou vissée dans le raccord (11, 71, 171), et pour le détachement de la cartouche de ciment osseux (10, 70) et/ou de la nouvelle cartouche de ciment osseux (10, 70) du raccord (11, 71, 171), la cartouche de ciment osseux (10, 70) ou la nouvelle cartouche de ciment osseux (10, 70) est tournée ou dévissée hors du raccord (11, 71, 171), et/ou la rotation du corps de soupape (6, 66, 166) par rapport au siège de soupape (3, 63, 163) est effectué par un vissage du corps de soupape (6, 66, 166) dans le siège de soupape (3, 63, 163) ou par une rotation manuelle du corps de soupape (6, 66, 166) par rapport au siège de soupape (3, 63, 163), la rotation manuelle étant effectuée de préférence par l'actionnement d'un levier (38, 98, 198) s'étendant radialement à partir du corps de soupape (6, 66, 166), lequel s'étend à travers un évidement (36, 96) dans le moule de coulée (1, 61) ou dans le siège de soupape (3, 63, 163).

15. Procédé selon l'une quelconque des revendications 12 à 14, **caractérisé en ce que** l'injection de la pâte de ciment osseux (50) de la cartouche de ciment osseux (10, 70) ou de la nouvelle cartouche de ciment osseux (10, 70) est effectuée par pression d'un piston (46, 106) dans un espace intérieur de la cartouche de ciment osseux (10, 70) et/ou avant l'étape B), et de préférence avant l'étape A), un noyau métallique (16, 76) est disposé à l'intérieur du moule de coulée (1, 61), le noyau métallique (16, 76) étant de préférence espacé d'une paroi intérieure du moule de coulée (1, 61) par plusieurs chevilles de maintien (18, 78), les plusieurs chevilles de maintien (18, 78) étant encore plus préférablement fixées dans des alésages (58) dans le noyau métallique (16, 76) et dans des cavités pour la réception de chevilles de maintien (18, 78) dans la paroi intérieure du moule de coulée (1, 61).
